(19) Europäisches Patentamt
European Patent Office
Office européen des brevets

(11) **EP 2 186 469 B1**

(12) **FASCICULE DE BREVET EUROPEEN**

(45) Date de publication et mention
de la délivrance du brevet:
**29.04.2015 Bulletin 2015/18**

(51) Int Cl.:
**A61B 5/00** (2006.01)

(21) Numéro de dépôt: **09175560.3**

(22) Date de dépôt: **10.11.2009**

(54) **Procédé et dispositif de localisation de fluorophores ou d'absorbeurs dans un milieu environnant**

Verfahren und Vorrichtung für die Lokalisierung von Fluorophoren oder Absorbern in einem umgebenden Medium

Method and device for the localisation of fluorophores or absorbers in surrounding medium

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **13.11.2008 FR 0857699**

(43) Date de publication de la demande:
**19.05.2010 Bulletin 2010/20**

(73) Titulaire: **Commissariat à l'Énergie Atomique et aux Énergies Alternatives 75015 Paris (FR)**

(72) Inventeurs:
• **Da Silva, Anabela**
**13004, Marseille (FR)**
• **Dinten, Jean-Marc**
**69008, Lyon (FR)**
• **Rizo, Philippe**
**38700, La Tronche (FR)**

(74) Mandataire: **Ilgart, Jean-Christophe et al BREVALEX 95, rue d'Amsterdam 75378 Paris Cedex 8 (FR)**

(56) Documents cités:
**US-A- 6 058 324**

• **AURÉLIE LAIDEVANT: "Méthodes optiques résolues en temps pour la tomographie de fluorescence dans les milieux diffusants" [Online] 27 décembre 2006 (2006-12-27), HYPER ARTICLES EN LIGNE , XP002532320 Extrait de l'Internet: URL:http://hal.archives-ouvertes.fr/ index. php? halsid=5s427klj0ls9ljhm9jf9rgpgf3&view _this_ doc=tel-00122185&version=1> * page 31 - page 32 * * page 117 - page 118 * * page 121 - page 127 * * page 131 - page 138 * * page 144 - page 147 ***

**Description**

## DOMAINE TECHNIQUE ET ART ANTÉRIEUR

L'invention concerne le domaine de l'imagerie optique diffuse sur les tissus biologiques par des méthodes résolues en temps.

Elle s'applique à la localisation de fluorophores ou d'absorbeurs dans un milieu diffusant tel que, par exemple, un organe d'un animal ou d'un être humain (cerveau, sein, tout organe où des fluorophores peuvent être injectés).

En particulier, un intérêt d'une molécule qui absorbe est le suivant. Certaines tumeurs cancéreuses sont visibles par le simple fait qu'elles présentent un coefficient d'atténuation plus important que les tissus sains. Dans ce cas, il est important de pouvoir identifier ces tumeurs.

Dans ce contexte, on peut également être amené à utiliser un fluorophore, qui est un marqueur fluorescent spécifique. Celui-ci vient se fixer de façon préférentielle sur les cellules cibles d'intérêt (par exemple des cellules cancéreuses). Un tel fluorophore offre un meilleur contraste de détection qu'un marqueur non spécifique. Les techniques optiques d'imagerie moléculaire de fluorescence ont pour but de localiser spatialement ces marqueurs fluorescents.

Les systèmes de tomographie optique utilisent diverses sources de lumière. Il existe donc des appareils qui fonctionnent en mode continu, d'autres en mode fréquentiel (qui utilisent des lasers modulés en fréquence) et enfin des appareils fonctionnant en mode temporel, qui utilisent des lasers pulsés.

Ainsi, il existe trois méthodes d'imagerie optique diffuse, qui se distinguent selon que la source de lumière utilisée soit continue, fréquentielle (i.e. dont l'intensité est modulée par une fonction sinusoïde du temps) ou pulsée.

Les instruments utilisant une source de lumière continue ont été les premiers à être utilisés, la source de lumière étant une source blanche filtrée ou une source monochromatique, telle un Laser. On utilise alors des détecteurs ponctuels ou bidimensionnels mesurant l'intensité de la lumière réfléchie ou transmise par un tissu éclairé par la source lumineuse.

La seconde catégorie d'imagerie optique diffuse, dite imagerie fréquentielle, utilise une source de lumière modulée en intensité à une fréquence donnée. La source lumineuse est le plus souvent une source laser modulée en intensité à des fréquences f généralement comprises entre quelques dizaines de kHz à quelques centaines de MHz. Le détecteur utilisé mesure à la fois l'amplitude du signal lumineux réfléchi ou transmis par le tissu, ainsi que la phase de ce signal lumineux par rapport à celle de la source de lumière.

Enfin, la troisième catégorie d'imagerie optique diffuse est l'imagerie optique diffuse pulsée, également appelée imagerie optique diffuse impulsionelle, ou encore temporelle, ou encore résolue en temps. La source utilisée produit des impulsions lumineuses dites impulsionnelles, c'est-à-dire de courte durée, à un taux de répétition donné. Les sources utilisées peuvent être des diodes laser pulsées picosecondes, ou des lasers femtosecondes. La durée de l'impulsion étant généralement inférieure à 1ns, on parlera alors de sources lumineuses pulsées subnanosecondes. Le taux de répétition est le plus souvent compris entre quelques centaines de kHz à quelques centaines de MHz.

L'invention concerne cette troisième catégorie d'imagerie de fluorescence.

Document WO2006/032151 divulgue un procédé et un système d'imagerie optique pour la localisation de fluorophores ou absorbeurs dans un tissue où une fonction d'étalement temporel est mesurée en plusieurs couples différents de positions de la source et du détecteur, des moments de ces curves sont calculés et utilisés pour déterminer la position dudit fluorophore ou absorbeur.

Les données temporelles sont celles qui contiennent le plus de contenu informationnel sur le tissu traversé, mais pour lesquelles les techniques de reconstruction sont les plus complexes. La mesure en chaque point d'acquisition est en effet une fonction dépendante du temps (appelée TPSF pour « Temporal Point Spread Function », ou fonction d'étalement temporel).

On cherche à extraire des paramètres simples de la TPSF, dont on connaît par ailleurs l'expression théorique. Ensuite, la résolution du problème inverse permet de retrouver la distribution des marqueurs fluorescents.

Dans certains cas, on cherche à localiser un fluorophore en vue d'une intervention ultérieure. A l'heure actuelle, la localisation de cellules malades peut se faire à l'aide des techniques de biopsie. Mais, afin de trouver la position, même approximative, de ces cellules, on doit effectuer plusieurs prélèvements. Cette technique invasive est évidemment délicate à mettre en oeuvre et est consommatrice de temps.

Il se pose donc le problème de la détermination de la localisation, même de manière approximative, d'un fluorophore ou d'un absorbeur fixé sur une zone d'un milieu, par exemple un milieu biologique, cela avec un nombre limité de mesures, et une durée de dépouillement relativement faible, voire proche du temps réel. L'invention trouve toute son utilité pour réaliser une telle détermination dans des milieux présentant une accessibilité limitée, par exemple lors du dépistage de tumeurs cancéreuses de la prostate à partir de mesures endoscopiques, ou lors du dépistage de cancers du sein, ou d'ostéosarchomes....

## EXPOSÉ DE L'INVENTION

L'invention concerne d'abord un procédé de localisation d'au moins un fluorophore ou d'au moins un élément (molécule notamment) absorbant - encore appelé absorbeur - dans un milieu diffusant, à l'aide d'une source de rayonnement apte à émettre un rayonnement d'excitation de ce fluorophore et des moyens de détection apte à mesurer :

- un signal de fluorescence ($\Phi_{fluo}$) émis par ce fluorophore,
- ou un signal réémis à la même longueur d'onde que celle d'excitation (issue de la source de rayonnement) par l'élément absorbant qui absorbe et diffuse le rayonnement absorbé. L'élément absorbant peut faire partie d'une zone d'un milieu qui présente un coefficient d'absorption supérieur à celui du milieu diffusant. On a déjà donné l'exemple de certaines tumeurs cancéreuses.

Ce procédé de localisation comporte les étapes suivantes:

a) pour au moins 3 couples différents de positions de la source de rayonnement et des moyens de détection, au moins une excitation par un rayonnement provenant de la source de rayonnement, et au moins une détection par les moyens de détection du signal de fluorescence émis par ce fluorophore après cette excitation, ou du signal d'émission émis par l'absorbeur ou par l'élément absorbant,

b) pour chacun de ces couples, l'identification d'une surface sur laquelle le fluorophore (ou l'élément absorbant) est situé, ou d'un volume comprenant cette surface et dans lequel le fluorophore ou l'absorbeur est situé,

c) une estimation de la localisation du fluorophore ou de la localisation de l'élément absorbant dans son milieu diffusant, par calcul de l'intersection des trois surfaces, ou éventuellement dans un volume autour de cette intersection.

Les signaux peuvent être détectés par exemple par technique TCSPC ou par caméra.

Un tel procédé peut être mis en oeuvre quelles que soient les positions respectives du fluorophore ou de l'absorbeur, de la source et du détecteur.

La précision du procédé ne sera pas la même suivant que l'on prend des positions de la source et des moyens de détection rapprochées ou éloignées du fluorophore ou de l'absorbeur. Mais le procédé permet dans tous les cas une localisation, même grossière, avec un faible nombre de mesures, avec un dépouillement rapide, voire en temps réel. Un tel procédé permet donc d'orienter la réalisation de biopsies ou d'examens non invasifs complémentaires.

En outre ce procédé est de mise en oeuvre très rapide, on peut réaliser une localisation d'un fluorophore ou d'absorbeur en moins d'une minute, ou même en moins de 15 s.

Pour chaque couple des au moins 3 couples différents, on peut définir un histogramme représentant le nombre de photons détectés en fonction du temps ; cet histogramme peut être appelé fonction temporelle de fluorescence.

La source de rayonnement ou de lumière et/ou les moyens de détection peuvent être respectivement l'extrémité d'une fibre optique qui amène la lumière d'excitation dans le milieu et/ou l'extrémité d'une fibre optique qui prélève une partie de la lumière d'émission. Pour la source, il peut également s'agir d'une source lumineuse de type Laser ou LED. En ce qui concerne le détecteur, il peut s'agir d'un tube photomultiplicateur, ou d'un capteur d'image.

Selon l'invention l'élément visé par la détection peut être un fluorophore unique, ou une pluralité ou une agrégation de fluorophores groupés sensiblement en un même lieu. Il peut également s'agir d'un absorbeur ou d'une pluralité d'absorbeurs groupés sensiblement en un même lieu (agrégation). Dans la suite du texte, un absorbeur sera assimilé à un fluorophore dont la longueur d'onde d'émission est égale à la longueur d'onde d'excitation, et dont la durée de vie est nulle. Autrement dit un milieu absorbant, ou un absorbeur, est un fluorophore dont la longueur d'onde d'émission est égale à la longueur d'onde d'excitation, et dont la constante de temps $\tau$ est nulle. On n'emploiera donc que l'expression « fluorophore », sauf lorsque des spécificités des absorbeurs sont expliquées.

Le volume autour de l'intersection des trois surface calculées peut résulter de l'intersection de trois volumes, chaque volume étant associé à l'une des surfaces et comportant l'ensemble des points de la surface et l'ensemble des points qui satisfont à l'équation de la surface avec une marge d'erreur, par exemple $\pm$ 10% ou $\pm$ 5%.

Le milieu diffusant environnant le fluorophore peut avoir toute forme de géométrie : par exemple, il est de type infini.

Il peut aussi être de type semi-infini, limité par une paroi. Un milieu semi infini, pour lequel chacune des extrémités de fibres d'amenée du signal d'excitation et de prélèvement du signal de fluorescence est disposée à plus de 1 cm, ou de 1,5 cm ou de 2 cm de la paroi qui limite ce milieu peut être traité, du point de vue du procédé selon l'invention, comme un milieu infini.

Le milieu diffusant peut encore former une tranche (géométrie dite « slab »), limitée par deux surface parallèles. L'invention s'applique aussi à un milieu de forme quelconque, dont la surface extérieure est décomposée en une série de plans.

Dans certains modes de réalisation, les surfaces à l'intersection desquelles se trouve le fluorophore, peuvent avoir la forme d'ellipsoïdes, mais il peut également s'agir de surfaces 3D n'ayant pas cette forme.

Selon un mode de réalisation, l'étape b) comporte pour chaque couple de position, un calcul d'un moment temporel normé, d'ordre n (n$\geq$ 1), d'une fonction temporelle de fluorescence. Un tel moment peut être obtenu par une dérivée d'ordre n d'une transformée de ladite fonction temporelle de fluorescence, par exemple une transformée de Fourier, ou une transformée de Laplace ou une transformée de Mellin.

On rappelle qu'un moment normé d'ordre n (n$\geq$ 1), noté $m_n$ d'une distribution g(t) est défini par

$$m_n = \frac{\int_{-\infty}^{+\infty} t^n g(t)dt}{\int_{-\infty}^{+\infty} g(t)dt}$$

Dans certains cas, il peut être avantageux de réaliser une excitation par une source de lumière, et une détection par les moyens de détection, pour 4 couples différents de positions de la source de rayonnement et des moyens de détection.

On peut ensuite :

- sélectionner parmi les quatre couples, le couple de points, dit quatrième couple de points, pour lequel la durée moyenne d'arrivée des photons, de l'émetteur au récepteur, est la plus courte, et calculer, pour ce quatrième couple de points, l'équation d'une surface sur laquelle le fluorophore est situé ;
- et, pour chacun des trois autres couples de points, après calcul d'une première équation d'une surface sur laquelle le fluorophore est situé, on peut effectuer une différence entre cette équation et celle associée au quatrième couple, pour obtenir une deuxième équation indépendante de la durée de vie du fluorophore.

On peut donc, selon un mode de réalisation, lors de l'étape b) pour chaque couple de position, calculer une équation de chaque surface, indépendante de la durée de vie du fluorophore, qui peut résulter de la différence entre le temps moyen calculé pour chaque couple de position et le temps moyen calculé pour un autre couple de position. Cet autre couple de position peut être celui ayant un temps moyen minimal ou ayant un temps moyen calculé minimal.

Un procédé selon l'invention peut en outre comporter une représentation visuelle ou graphique de la position ou de la répartition du ou des fluorophores ou absorbeurs.

L'invention concerne également un procédé pour localiser une zone dans un milieu, comportant l'introduction dans ce milieu d'au moins un fluorophore ou d'un absorbeur, et la localisation de ce fluorophore ou de cet absorbeur dans ledit milieu par un procédé selon l'invention. La zone peut être par exemple un milieu constitué d'un organe humain ou animal, le fluorophore ou l'absorbeur permettant de marquer cette zone. Le but peut être de localiser des cellules malades dans un tel milieu. Comme déjà indiqué ci-dessus, l'invention permet de réaliser cette localisation en un temps rapide, ou même en temps réel.

Un procédé selon l'invention peut être mis en oeuvre de manière invasive ou non invasive.

L'invention concerne également un dispositif pour mettre en oeuvre un procédé selon l'invention, et permettant la localisation d'un fluorophore dans un milieu diffusant.

L'invention concerne donc également un dispositif pour la localisation d'un fluorophore dans un milieu diffusant, qui peut être de l'un des types évoqués plus haut, comportant :

- au moins une source de lumière ou de rayonnement, apte à émettre un rayonnement d'excitation de ce fluorophore,
- au moins un moyen de détection apte à mesurer un signal de fluorescence ($\Phi_{fluo}$) émis par ce fluorophore,
- des moyens de calcul, programmés pour calculer :

   a) pour chaque couple de points parmi au moins 3 couples différents de positions d'une source de rayonnement et d'un moyen de détection, la détermination, après une excitation réalisée par un rayonnement provenant de la source de rayonnement, et une détection par les moyens de détection des signaux de fluorescence émis par ce fluorophore après cette excitation, d'une surface sur laquelle le fluorophore est situé, ou un volume comprenant cette surface et dans lequel le fluorophore est situé,

   b) une estimation de la localisation du fluorophore dans son milieu environnant, par calcul de l'intersection des trois surfaces ou d'un volume autour de cette intersection.

Le milieu environnant peut être de type infini ou de type semi-infini ou en forme une tranche, limitée par deux surfaces parallèles, ou de forme quelconque, sa surface extérieure étant décomposée en une série de plans.

Les moyens de calcul, par exemple des moyens électroniques tels qu'un micro ordinateur ou un calculateur ou un microprocesseur, sont programmés pour mémoriser et traiter des données de fluorescence ou d'émission obtenues des moyens de détection. De préférence ces moyens sont programmés pour mettre en oeuvre un procédé de traitement selon l'invention.

Les moyens de calcul permettent de calculer, pour chaque couple de position, un moment temporel normé, d'ordre n (n $\geq$ 1), d'une fonction temporelle de fluorescence. Ce moment peut être obtenu à partir d'une fonction fréquentielle, déduite de ladite fonction temporelle de fluorescence par exemple par transformée de Fourier ou par transformée de Mellin.

Selon un mode avantageux, les moyens de calcul permettent ou sont programmés pour réaliser, pour la localisation d'au moins un fluorophore ou absorbeur, et pour chaque couple de position, un calcul d'une équation de chaque surface, indépendante de la durée de vie du fluorophore. A cette fin, les moyens de calcul permettent de réaliser la différence entre le temps moyen calculé pour chaque couple de position et le temps moyen mesuré ou calculé pour un autre couple de position.

Le dispositif permet une excitation par la source de rayonnement, et une détection par les moyens de détection pour 4 couples différents de positions de la source de rayonnement et des moyens de détection.

Le quatrième couple de position peut être un couple pour lequel le signal de fluorescence a un temps moyen mesuré ou un temps moyen calculé inférieur à celui des 3 couples de position.

## BRÈVE DESCRIPTION DES DESSINS

- Les figures 1A et 1B représentent chacune un exemple de dispositif expérimental pour la mise en oeuvre de l'invention,
- les figures 2A et 2B représentent respectivement une série d'impulsions laser et de photons uniques émis, et une courbe de fluorescence obtenue à partir des données relatives aux photons uniques,
- la figure 3 représente une configuration théorique, en deux dimensions,
- les figures 4A-4F représentent le dispositif expérimental d'une mesure réalisée, le positionnement des points source et de détection, des courbes de fluorescence mesurées, et la position calculée et la position réelle d'un fluorophore;
- la figure 5A représente des couples de positions d'une source et d'un détecteur, dans un plan XY, en vue d'une mesure selon l'invention, en configuration semi-infinie,
- les figures 5B-5D représentent des portions d'ellipsoïdes utilisés dans la cadre d'une mesure selon l'invention, en configuration semi-infinie,
- les figures 6A-6C représentent chacune une localisation d'un fluorophore obtenue avec un procédé selon l'invention, en configuration semi-infinie,
- les figures 7A - 7D représentent des surfaces mises en oeuvre dans le cadre d'un autre mode de réalisation de l'invention,
- les figures 8A-8D représentent des portions de surfaces utilisées dans la cadre d'une mesure selon l'invention, en configuration de tranche.
- la figure 9 représente un schéma de déroulement d'un procédé selon l'invention.
- les figures 10A-10D représentent diverses dispositions, par rapport à un milieu étudié, de moyens d'excitation et de détection, ou de fibres optiques amenant à ce milieu un rayonnement d'excitation et transmettant un rayonnement à détecter.

## EXPOSÉ DÉTAILLÉ DE MODES DE RÉALISATION PARTICULIERS

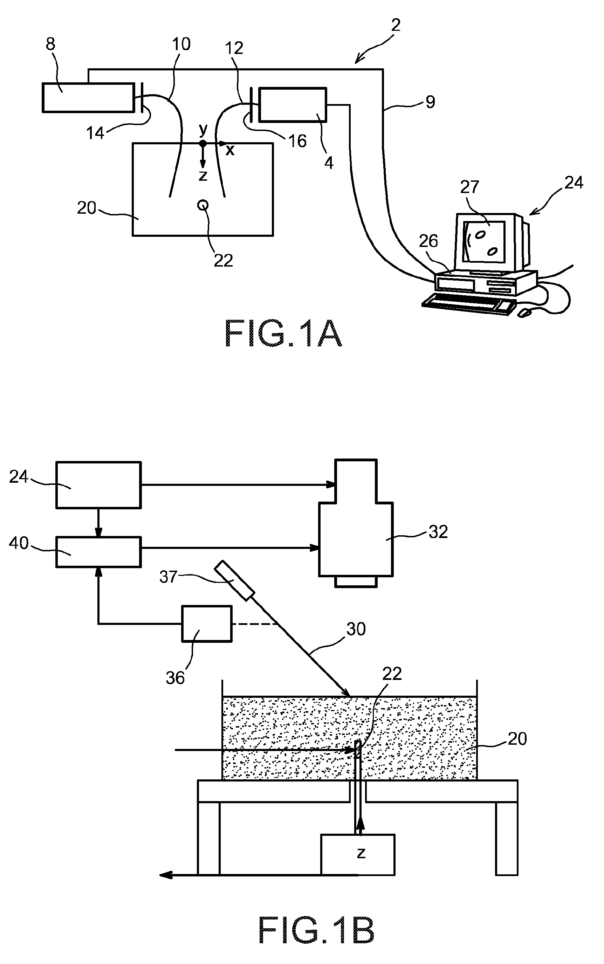

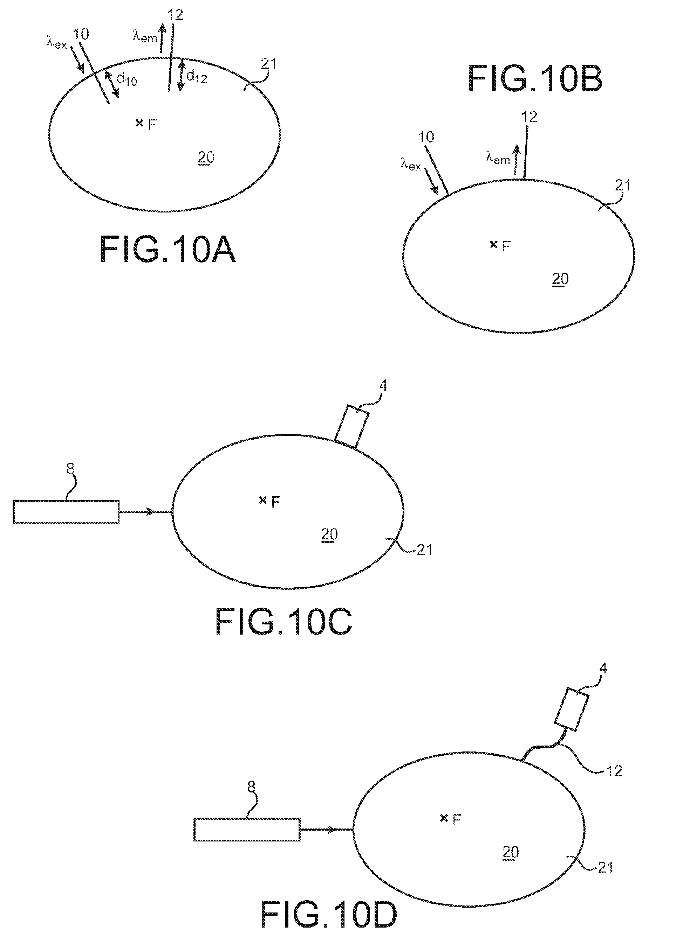

La figure 1A est un exemple de système expérimental 2 pour mettre en oeuvre un procédé selon l'invention. Des représentations schématiques d'un milieu et de moyens d'excitation et de détection sont donnés plus loin en liaison avec les figures 10A-10D.

Le dispositif de la figure 1A utilise comme détecteur 4 un photomultiplicateur et une carte TCSPC (Time Correlated Single Photon Counting), en fait intégrée dans un ensemble de moyens 24 de traitement de données.

La lumière est émise par une source 8 de rayonnement en impulsions. La durée de chaque impulsion est généralement inférieure à 1ns, par exemple comprise entre d'une part quelques centaines de fs, par exemple 500 fs, ou 100 ps ou 500 ps et d'autre part 100 ps ou 500 ps ou 1 ns. Le taux de répétition est de préférence compris entre quelques centaines de kHz, par exemple 100 kHz ou 500 kHz et quelques centaines de MHz, par exemple 100 MHz ou 500 MHz.

La lumière émise par la source 8 est envoyée et collectée par des fibres 10, 12 qui peuvent être déplacées.

Les deux fibres peuvent être montées sur des moyens de déplacement en translation verticaux et horizontaux (suivant les axes X, Y et Z de la figure 1A). Pour cela on peut mettre en oeuvre une ou des platines montées en translation pour déplacer les extrémités des fibres optiques. On peut aussi utiliser un laser, lui aussi monté sur une platine montée en translation. Un détecteur peut lui aussi être monté sur une telle platine. Dans tous les cas la translation peut être pilotée par ordinateur.

La source 8 d'impulsions de rayonnement peut également être utilisée comme moyen de déclenchement de la carte TCSPC (voir la liaison 9 entre la source 8 et les moyens 24). Il est également possible de travailler avec des impulsions dans le domaine de la femto seconde, à condition de disposer de la source de rayonnement adéquate, c'est-à-dire d'une source laser 8 dont chaque impulsion a une largeur temporelle également dans le domaine de la femto seconde.

Selon un mode particulier de réalisation, la source 8 est une diode laser pulsée, par exemple à une longueur d'onde voisine de 630 nm et avec un taux de répétition d'environ 50 MHz.

La lumière laser passe de préférence par un filtre interférentiel 14 pour éliminer d'éventuels modes secondaires.

La fibre 12 collecte la lumière en provenance du milieu 20 étudié, en particulier la lumière diffusée par ce milieu. Un filtre interférentiel 16 et/ou un filtre coloré absorbant les faibles longueurs d'onde peuvent être placés devant le détecteur 4 pour sélectionner la lumière de fluorescence (par exemple : $\lambda > 650$ nm, la source étant à une longueur d'onde de, par exemple, 631 nm) d'un fluorophore 22 disposé dans le milieu 20 et optimiser l'élimination de la lumière d'excitation.

Dans le texte de la présente demande, quel que soit le mode de réalisation de l'invention, il sera notamment question de la position de la source de rayonnement, et/ou de la position des moyens de détection. Lorsque des fibres sont utilisées, ces positions s'entendent le plus souvent comme celles des extrémités des fibres qui amènent le rayonnement dans le milieu diffusant 20, ou à l'interface de ce milieu, et /ou celles qui collectent le rayonnement diffusé, ces dernières étant placées dans le milieu ou à l'interface de ce milieu, mais ne s'entendent alors pas comme étant celles de la source 8 proprement dite ou du détecteur 4 proprement dit.

En variante, on peut utiliser une source de lumière délivrant un faisceau lumineux, par exemple un laser en émission, et une caméra munie d'un objectif en sortie, ce qui permet un couplage optique entre la face sensible de la caméra et la face de sortie de l'objet observé (ce qui est le cas du dispositif de la figure 1B).

En application de type « endoscopie », on utilise préférentiellement un système à fibres, les fibres pouvant être par exemple intégrées dans une sonde endo-rectale, au moins une fibre transmettant la source de lumière pulsée d'excitation à la zone à examiner. Cette source de lumière pulsée peut être externe à la sonde. Au moins une autre fibre transmet le signal optique d'émission de la zone à examiner vers un photodétecteur, qui peut lui aussi être externe à la sonde. Selon la technique TCSPC (pour « Time Correlated Single Photon Counting », ou comptage de photons uniques correlés en temps) on détecte, à l'aide du photomultiplicateur, un photon émis par le fluorophore après une impulsion de la source de rayonnement.

Le système permet donc une détection résolue en temps des impulsions de fluorescence. Il permet de récupérer des photons de fluorescence.

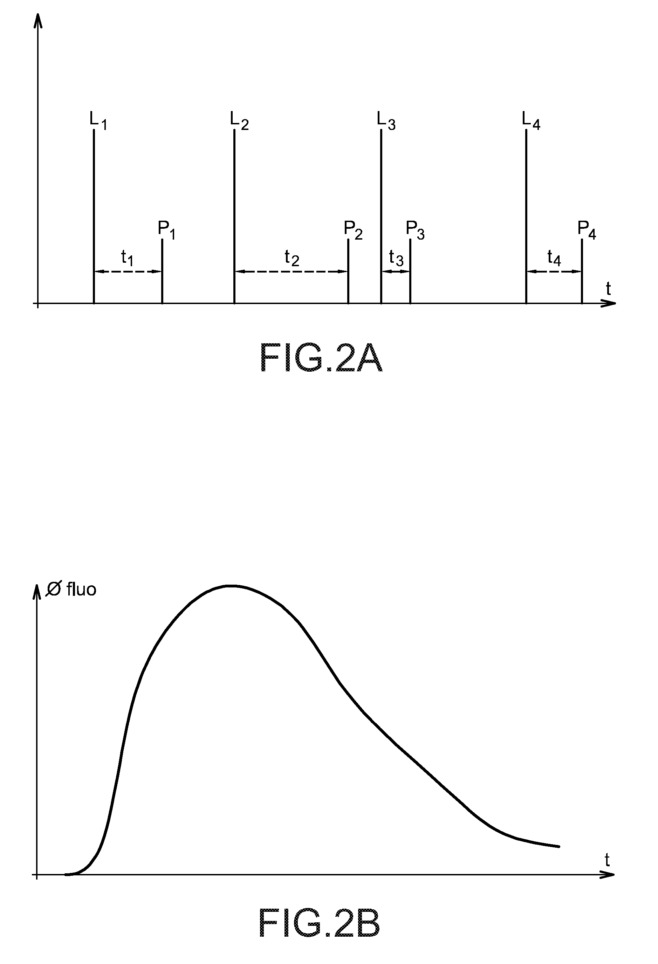

La figure 2A représente une série d'impulsions laser Li (i = 1 - 4) et une série de photons uniques pi (i = 1 - 4) correspondants, ces derniers étant détectés par un système de type TCSPC (Time Correlated Single Photon Counting). Chaque photon est en fait détecté par rapport au départ de l'impulsion correspondante : sur la figure 2A, ti représente la durée écoulée entre chaque impulsion laser Li et l'instant de détection de chaque photon pi.

Il est donc ensuite possible d'établir une répartition statistique ou histogramme du temps d'arrivée des photons, comme illustré sur la figure 2B, qui représente le nombre de photons de fluorescence détectés, en fonction du temps écoulé t par rapport à chaque impulsion laser. On peut déterminer, à partir d'un tel histogramme, des paramètres statistiques, notamment une moyenne du temps d'arrivée, ou temps moyen (il s'agit en fait de la moyenne des abscisses pondérée par les ordonnées de l'histogramme).

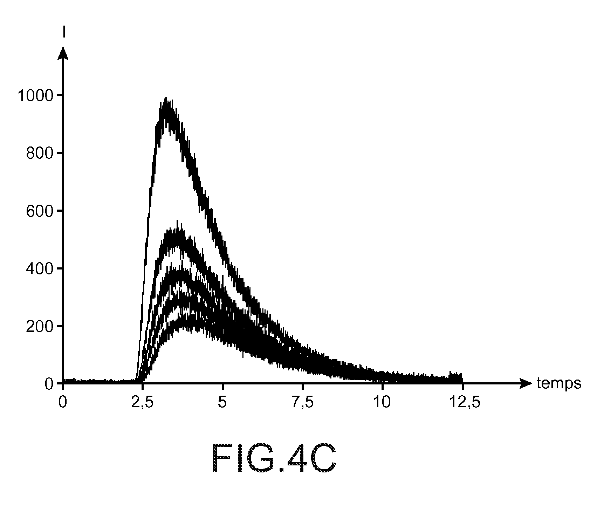

Une telle courbe $\Phi_{fluo}$ (t) qui, on le voit (voir également l'exemple de la figure 4C), permet d'utiliser toute l'information sur une large fenêtre temporelle, de part et d'autre du point d'intensité maximum (et pas seulement dans la partie montante du signal) peut donc ensuite être traitée pour en tirer des informations caractéristiques, tel que le temps d'arrivée, ou temps moyen comme on va l'expliquer ci-dessous.

Des moyens électroniques 24 tels qu'un micro ordinateur ou un calculateur sont programmés pour mémoriser et traiter les données de la carte TCSPC. Plus précisément une unité centrale 26 est programmée pour mettre en oeuvre un procédé de traitement selon l'invention. Des moyens 27 d'affichage ou de visualisation permettent, après traitement, de représenter le positionnement du ou des fluorophores dans le milieu 20 examiné.

D'autres techniques de détection peuvent être employées, par exemple avec une caméra intensifiée, et par exemple une caméra intensifiée ultra-rapide de type « gated camera » ; dans ce cas la caméra s'ouvre sur une porte temporelle, de largeur par exemple environ 200 ps, puis cette porte est décalée, par exemple de 25 ps en 25 ps.

La figure 1B est un exemple d'un autre système expérimental 2 utilisant comme détecteur 32 une caméra rapide : la position relative de la source (ou le détecteur) et l'objet peut aisément être réalisé.

Un faisceau 30 d'excitation de la fluorescence d'un milieu 20, contenant un ou plusieurs fluorophores 22, est émis par une source 37 de rayonnement (non représentée sur la figure) qui peut être du même type que celle présentée ci-dessus en liaison avec la figure 1A. Un photodétecteur 36 permet de commander des moyens 40 formant une ligne à retard. La référence 24 désigne, comme sur la figure 1A, des moyens électroniques de traitement de données de type micro-ordinateur ou calculateur, programmés pour mémoriser et traiter les données de la caméra 32. Une unité centrale de ces moyens 24 est programmée pour mettre en oeuvre un procédé de traitement selon l'invention. Là encore, des moyens d'affichage ou de visualisation permettent, après traitement, de représenter le positionnement ou la localisation d'un ou de plusieurs fluorophores dans le milieu 20 examiné.

La distance entre le faisceau lumineux émis par la source, et le détecteur, ou la partie du milieu optiquement couplée au détecteur, est par exemple comprise entre quelques mm et quelques cm, par exemple entre 1 mm et 20 cm dans le cas de milieux biologiques. C'est le cas notamment dans le cas d'une intégration dans une sonde endo-rectale. Le positionnement relatif de la source et du détecteur par rapport à un organe à examiner peut être déterminé par des moyens de type optique ou ultrasonore, par exemple une sonde d'echographie.

En variante des dispositifs ci dessus, on peut placer un nombre n de sources lumineuses et un nombre p de détecteurs, avec par exemple $1 \leq n \leq 3$ et $1 \leq p \leq 3$, les sources lumineuses et les détecteurs pouvant être des extrémités de fibres optiques reliées respectivement à au moins une source de lumière pulsée ou au moins un photodétecteur, de type photomultiplicateur ou caméra. On combine alors ces positions de manière à avoir au moins 3 couples distincts de positions de la source et de positions de détecteur :

- une position de source S1 (cas n = 1) successivement avec 3 positions distinctes D1, D2, D3 de détecteur (cas p=3) ; donc on utilise les couples de positions (S1, D1), (S1, D2), (S1, D3),
- deux positions distinctes S1 et S2 de la source (cas n = 2) avec 2 ou 3 positions distinctes de détecteur (cas p=2 ou 3) ; donc on utilise par exemple les couples de positions (S1, D1), (S2, D2) et (S1, D2) ou (S1, D1), (S2, D2) et (S1, D3),
- trois positions distinctes S1, S2 et S3 de la source (cas n = 3) avec 1 ou 2 positions distinctes de détecteur (cas p=1 ou 2) ; donc on utilise par exemple les couples de positions (S1, D1), (S2, D1) et (S3, D1) ou (S1, D1), (S2, D2) et (S3, D2).

De préférence on utilise 3 couples pour lesquels les 3 positions de source sont distinctes entre elles et les 3 positions de détecteur sont distinctes entre elles (configuration : (S1, D1), (S2, D2) et (S3, D3)).

La lumière d'excitation, à la longueur d'onde $\lambda_x$ excite le fluorophore qui réémet une lumière dite d'émission à la longueur d'onde $\lambda_m > \lambda_x$ avec une durée de vie $\tau$. Cette durée de vie correspond à la durée moyenne pendant laquelle les électrons excités restent dans cet état avant de retourner à leur état initial. Les développements qui vont suivre montrent que le cas d'un absorbeur, et donc d'une émission à longueur d'onde sensiblement égale à celle d'excitation avec une durée de vie nulle, peut être déduit de cas d'un fluorophore.

L'équation de propagation de la lumière d'excitation dans un milieu diffusant produite par la source pulsée de rayonnement s'écrit :

$$\frac{1}{c_n}\frac{\partial \phi_x(\mathbf{r},t)}{\partial t} + \vec{\nabla}.(-D_x(\mathbf{r})\nabla\phi_x(\mathbf{r},t)) + \mu_{ax}(\mathbf{r})\phi_x(\mathbf{r},t) = S_x(\mathbf{r}_s,t) \qquad (1)$$

où

- $r_s$ désigne la position de la source,
- r désigne une position quelconque dans le milieu,
- la fonction $\Phi_x(\mathbf{r}, t)$ désigne le débit de fluence du rayonnement d'excitation au point r à l'instant t.
- le coefficient $D_x(r)$ est le coefficient de diffusion au point r, à la longueur d'onde d'excitation $\lambda_x$ ;
- $S_x(\mathbf{r}_s, t)$ désigne le flux de rayonnement émis, à l'instant t, par la source au point $r_s$.

Celle de la lumière d'émission s'écrit :

$$\frac{1}{c_n}\frac{\partial \phi_m(\mathbf{r},t)}{\partial t} + \vec{\nabla}.(-D_m(\mathbf{r})\nabla\phi_m(\mathbf{r},t)) + \mu_{am}(\mathbf{r})\phi_m(\mathbf{r},t) = \frac{1}{\tau}\eta\mu_{ax-->m}\int_0^t \exp(\frac{-(t-t')}{\tau})\phi_x(\mathbf{r},t')dt'$$

$$(2)$$

avec :

- la fonction $\Phi_m(\mathbf{r}, t)$ qui désigne le débit de fluence du rayonnement d'émission au point r, à l'instant t ;
- le coefficient $D_m(r)$, qui est le coefficient de diffusion au point r à la longueur d'onde d'émission $\lambda m$.

Les conditions aux limites étant les suivantes:

$$\phi_x(\mathbf{r},t) + 2AD_x(\mathbf{r})\frac{\partial \phi_x}{\partial n}(\mathbf{r},t) = 0 \qquad (3)$$

$$\phi_m(\mathbf{r},t) + 2AD_m(\mathbf{r})\frac{\partial \phi_m}{\partial n}(\mathbf{r},t) = 0 \qquad \mathbf{(4)}$$

et ce pour tout point **r** de la frontière $\delta\Omega$ du milieu diffusant.

Ces équations sont aussi développées et expliquées dans la thèse de d'Aurélie Laidevant, « Méthodes optiques résolues en temps pour la tomographie de fluorescence dans les milieux diffusants », Université Joseph Fourier, Grenoble, 5 octobre 2006, p.51 et suivantes.

Dans ces équations :

- $\mu_{ax}$ et $\mu_{am}$ sont les coefficients d'absorption du milieu diffusant à la longueur d'onde d'excitation $\lambda_x$ et d'émission $\lambda_m$ respectivement,
- $\mu_{ax \rightarrow m}$ est le coefficient d'absorption associé à la concentration locale de fluorophores à la longueur d'onde d'excitation $\lambda_x$;
- $\eta$ est le rendement quantique du fluorophore,
- $\Phi(r,t)$ est le débit de fluence associé à la densité de photons, en photons/m$^2$s, ou, de manière équivalente, en Watt/m$^2$,
- A est égal à (1-R$_{eff}$)/(1+R$_{eff}$), où R$_{eff}$ est le coefficient de réflexion dû à la différence d'indice entre l'air et le milieu 22 environnant le fluorophore.

La condition aux limites exprime que, à la frontière du milieu, le débit de fluence diffusé est nul.

La fonction théorique $\Phi_m$ peut-être considérée comme proportionnelle à la TPSF, cette dernière étant mesurée expérimentalement (figure 2B). Ainsi, les moments temporels normés de la TPSF sont égaux aux moments temporels normés de la fonction $\Phi_m$.

La résolution du système d'équations couplées obtenu n'est pas aisée, et l'exploitation du volume de données que contient le signal temporel est difficile. On s'intéresse en général à l'exploitation de tel ou tel intervalle de temps extrait de ces fonctions dépendantes du temps (appelées TPSF pour « Temporal Spread Function », ou fonction d'étalement temporel)- pour réaliser une étape d'ajustement ('fitting') avec les données expérimentales.

Pour exploiter l'information temporelle par le biais d'autres grandeurs, extraites des TPSF mesurées, on peut définir d'autres mesures telles que la mesure des moments - transformées de Mellin de la TPSF, ou des Transformées de Fourier (et donc travailler en mode fréquentiel), ou des Transformées de Laplace ou par paramétrisation de la TPSF (c'est-à-dire la détermination de paramètres caractéristiques tels que les coordonnées du maximum, et/ou le coefficient de pente montante et/ou de pente descendante de la TPSF. L'intérêt de telles grandeurs est de réduire le volume de données en supprimant les éventuelles redondances existant entre elles

Dans la suite de l'invention, on s'intéresse principalement au calcul de moments d'ordre n de l'histogramme, un tel calcul pouvant être réalisé par transformée de Fourier.

En particulier, on peut, partant de la fonction $\Phi_m$, en calculer tout moment temporel normé, et notamment le moment d'ordre 1 normé. Une telle grandeur correspond au temps moyen d'arrivée des photons. Les moments temporels normés de la fonction $\Phi_m$, sont définis comme :

$$m_k = \int_{-\infty}^{+\infty} t^k \phi_m(r,t)dt \Big/ \int_{-\infty}^{+\infty} \phi_m(r,t)dt \qquad (6)$$

Le moment d'ordre 0 est:

$$m_o = \int_{-\infty}^{+\infty} \phi_m(r,t)dt$$

On va maintenant traiter le cas d'un milieu infini. On peut montrer que le moment temporel normé d'ordre 1 de la fonction $\Phi_m$, définissant le temps moyen d'arrivée des photons $<t>\infty$, en milieu infini s'écrit :

$$m_1 = \int_{-\infty}^{+\infty} t\phi_m(r,t)dt \Big/ \int_{-\infty}^{+\infty} \phi_m(r,t)dt = <t>_\infty = <t>_x + <t>_m + <t>_{déclin\ fluo}$$

$$(7)$$

$$= \frac{r_{sr}}{2c_n\sqrt{\mu_{ax}D_x}} + \frac{r_{rd}}{2c_n\sqrt{\mu_{am}D_m}} + \tau$$

où $<t>_x$, $<t>_m$ et $<t>_{déclin\ fluo}$ désignent respectivement la durée moyenne du trajet source-fluorophore, la durée du trajet fluorophore -détecteur, et la durée de vie moyenne de l'espèce excitée (cette dernière étant égale à 0 en présence d'un absorbeur).On rappelle que, en outre :

- $r_{sr} = |r_s - r|$
- $r_{rd} = |r-r_d|$
- $m_1$ est le résultat de la mesure

On voit qu'on a là l'équation d'une surface 3D, ayant ici la forme d'un ellipsoïde.

Plus généralement, le moment normé d'ordre k peut s'exprimer sous la forme :

$$m_k = (i)^k \frac{\partial^k \widetilde{\Phi}(\omega)}{\partial \omega^k}\Big|_{\omega = 0} \times \frac{1}{\widetilde{\Phi}(\omega)|_{\omega = 0}} \qquad (8)$$

Où $\widetilde{\Phi}(\omega)$ est la transformée de Fourier de $\Phi_m$:

$$\widetilde{\Phi}(\omega) = \frac{1}{\sqrt{2\pi}}\int_{-\infty}^{+\infty} \phi(t)e^{-i\omega t}dt \qquad \frac{\partial \widetilde{\Phi}(\omega)}{\partial \omega} = \frac{-i}{\sqrt{2\pi}}\int_{-\infty}^{+\infty} t\phi(t)e^{-i\omega t}dt$$

Par exemple, on peut montrer que la variance de la distribution temporelle s'écrit

$$V = <(t-<t>)^2> = m_2 - m_1^2, \text{ soit en milieu infini } V = \frac{r_{sr}}{2c_n^2\sqrt{\mu_{ax}^3 D_x}} + \frac{r_{rd}}{2c_n\sqrt{\mu_{am}^3 D_m}} + \tau^2.$$

On observe qu'on obtient donc, également dans le cas de la variance, la définition d'une surface tridimensionnelle, ici, celle d'un ellipsoïde.

Cette grandeur (la variance) peut être calculée dans le cadre de la présente invention à partir des moments d'ordre 2 et 1.

Plus généralement encore on peut, selon l'invention, calculer tout moment d'ordre supérieur à 1, et établir une relation liant au moins un de ces moments aux distances respectives source-fluorophore et fluorophore-détecteur. Si l'on considère des moments d'ordre supérieur, l'équation reliant ces derniers, ou une combinaison de ces derniers, aux distances respectives source-fluorophore et fluorophore-détecteur, est également celle d'une surface tridimensionnelle.

Préférentiellement, on utilise le moment d'ordre 1 normé.

D'une manière générale, on peut voir un fluorophore comme capable d'absorber une certaine quantité de lumière (dans son état excité) et d'en réémettre une partie à une longueur d'onde supérieure (lors de sa désexcitation), avec un retard correspondant à la durée dans l'état excité. A partir des expressions données ci dessus on peut donc passer du cas du fluorophore à l'absorbeur simple en écrivant $\mu_{ax} = \mu_{am}$, $D_x = D_m$ et $\tau = 0$. Ceci vaut pour tous les cas exposés dans la présente demande. Les considérations ci-dessus valent donc également pour le cas d'un objet absorbant.

Le milieu infini est typiquement celui que l'on rencontre lorsque des mesures invasives sont réalisées, avec des fibres 10, 12 pour amener la lumière dans un milieu 21 (cas de la figure 10A) et pour prélever le signal de fluorescence émis par le fluorophore F, les extrémités des fibres étant positionnées dans le milieu diffusant, chacune à une distance $d_{10}$ et $d_{12}$ d'au moins 1 cm ou 1,5 cm d'une paroi 21 qui délimite le milieu. On peut plus généralement considérer qu'on se trouve dans un milieu infini lorsque la source et le détecteur sont placés à une profondeur suffisante dans le milieu diffusant. En pratique, cela correspond à des mesures invasives, réalisées en disposant par exemple des fibres optiques, ou une partie de ces fibres, à l'intérieur d'un milieu diffusant.

On considère une première hypothèse, celle d'un milieu où les propriétés optiques sont identiques aux 2 longueurs d'onde d'excitation et de mesure, $\lambda_x$ et à $\lambda_m$, ce qui est par exemple sensiblement le cas lorsque ces deux longueurs d'onde sont distantes de par exemple de 10 nm ou de quelques dizaines de nm. Rappelons aussi que λx λm lorsqu'on n'a qu'un absorbeur non fluorescent. ; Les propriétés optiques du milieu aux deux longueurs d'ondes peuvent donc être considérées comme identiques. On a alors une simplification de l'équation (7) ci-dessus :

$$r_{sr} + r_{rd} = 2c_n \sqrt{\mu_a D}(<t>_\infty - \tau) \qquad (9)$$

Ceci constitue la formule d'un ellipsoïde de foyers S et D, soit le lieu des points r tels que la distance source-fluorophore $r_{sr}$ plus la distance fluorophore-détecteur $r_{rd}$ est égale au produit de la vitesse apparente dans le milieu diffusant $2c_n \sqrt{\mu_a D}$ multiplié par le temps moyen d'arrivée des photons corrigé du retard introduit par le temps de vie $\tau$.

Ainsi pour trouver la position 3D du fluorophore, il suffit d'effectuer 3 mesures à trois positions de sources et 3 positions de détecteurs. Le fluorophore est donc à l'intersection de 3 ellipsoïdes définis dans l'espace par 3 équations différentes. De préférence, on sélectionne des couples de positions définissant des directions sensiblement perpendiculaires les unes aux autres. Pour chaque couple, on procède à l'établissement d'une TPSF ou d'un histogramme comme expliqué ci-dessus.

Dans la deuxième hypothèse, le cas où les propriétés optiques ne peuvent pas être considérées identiques aux 2 longueurs d'onde, l'équation (7) devient:

$$\frac{r_{sr}}{v_x} + \frac{r_{rd}}{v_m} = <t>_x + <t>_m = (<t>_\infty - \tau)$$
$$v_{x,m} = 2c_n \sqrt{\mu_{ax,m} D_{x,m}} \qquad (10)$$

Ceci est toujours l'équation d'un ellipsoïde mais il traduit maintenant le lieu des points r tels que la distance source-fluorophore $r_{sr}$ soit « pondérée » par la vitesse apparente $v_x$ (d'où le terme $r_{sr}/v_x$), et la distance fluorophore-détecteur $r_{rd}$ soit « pondérée » par la vitesse apparente $v_m$ (d'où le terme $r_{rd}/v_m$).

Là encore, pour trouver la position 3D du fluorophore, on effectue 3 mesures à trois positions de sources et 3 positions de détecteurs. Le fluorophore est donc à l'intersection de 3 ellipsoïdes définis dans l'espace par 3 équations différentes. De préférence, on sélectionne des couples de positions définissant des directions sensiblement perpendiculaires les unes aux autres. Pour chaque couple de positions (source, détecteur), on procède à l'établissement d'une TPSF ou d'un histogramme comme expliqué ci-dessus. Egalement de préférence, lors de chaque acquisition, on tentera d'augmenter le plus possible la distance source-détecteur.

Les expressions des temps moyens peuvent être données analytiquement pour des géométries autres que celles du milieu infini, plus complexes : milieu semi-infini, avec une interface plane ; milieu plan à faces parallèles...

De manière générale, si l'on connait l'expression, analytique ou numérique, de la transformée de Fourier $\tilde{\Phi}(\omega)$ de la fonction $\phi_m$, alors :

$$m_1 = <t> = i\frac{\partial \tilde{\Phi}(\omega)}{\partial \omega}\Big|\omega = 0 \times \frac{1}{\tilde{\Phi}(\omega)\big|\omega = 0} \, ,$$

où

$$\widetilde{\Phi}(\omega) = \frac{1}{\sqrt{2\pi}} \int_{-\infty}^{+\infty} \phi(t) e^{-i\omega t} dt \ , \qquad \frac{\partial \widetilde{\Phi}(\omega)}{\partial \omega} = \frac{-i}{\sqrt{2\pi}} \int_{-\infty}^{+\infty} t\phi(t) e^{-i\omega t} dt$$

La surface décrite n'est plus un ellipsoïde, mais reste une surface 3D ; Donc, là encore, on localise un fluorophore par recherche des intersections entre 3 surfaces, chacune obtenue pour un couple de point (position source, position mesure) différent.

D'une manière générale, dans tous les cas concernés par l'invention (ceux-ci-dessus et les autres exposés ci-dessous), pour trouver la position 3D du fluorophore, on effectue 3 mesures avec trois positions, de préférence différentes entre elles, de sources et 3 positions, de préférence différentes entre elles, de détecteurs (autrement dit, une mesure pour chacun des 3 couples ($r_{si}$, $r_{di}$), i= 1,2,3, avec $r_{si} \neq r_{sj}$, pour i $\neq$ j et $r_{di} \neq r_{dj}$, pour i $\neq$ j). Le fluorophore est donc à l'intersection de 3 ellipsoïdes définis dans l'espace par 3 équations différentes. De préférence, on sélectionne 3 couples de positions définissant des directions sensiblement perpendiculaires les unes aux autres. Dans certains cas, dont on verra un exemple plus loin, il peut être utile d'effectuer 4 mesures pour 4 couples de positions (source, détecteur). Pour chaque couple de positions (source, détecteur), on procède à l'établissement d'une TPSF ou d'un histogramme comme expliqué ci-dessus.

Un autre exemple est le cas d'un milieu semi-infini. C'est le cas de tout organe sondé en surface, par exemple prostate, sein, cerveau, dont l'épaisseur est suffisamment grande devant les distances caractéristiques source-détecteur.

Ce cas est celui que l'on rencontre lorsque des mesures sont réalisées, avec des fibres 10, 12 pour amener la lumière et pour prélever le signal de fluorescence, les extrémités des fibres étant positionnées à la surface d'une paroi 21 qui délimite le milieu (figure 10B). Plus généralement, il s'agit du cas où source et détecteur sont positionnés à la surface de cette paroi 21 qui délimite le milieu diffusant dans lequel le fluorophore F est disposé

C'est notamment le cas où on souhaite être non invasif, source et détecteur étant positionnés à proximité de la surface du milieu. Ainsi, dans le cas d'un diagnostic d'une tumeur de la prostate, source et détecteur seront placés en périphérie d'une sonde endorectale.

Il s'agit dans ce cas de vérifier non seulement l'équation de propagation mais également les conditions aux limites associées, déjà indiquées ci-dessus :

$$\phi(\mathbf{r},t) + 2AD(\mathbf{r}) \frac{\partial \phi}{\partial n}(\mathbf{r},t) = 0 \quad ,$$

pour tout point r de la frontière $\delta\Omega$ du milieu diffusant, et cela pour les longueurs d'onde d'excitation et d'émission.

On montre que cette condition est équivalente à écrire, de manière plus simple, $\phi(\mathbf{r},t)=0 \ \forall \mathbf{r} \in \partial\Omega_{extrapolée}$, où la frontière extrapolée est située à une distance $d_{extrapolée}$ = 2AD de la frontière physique, comme expliqué dans l'article de A.Laidevant et al. « Effects of the surface boundary on the determination of the optical properties of a turbid medium with time resolved reflectance", Applied Optics, 45, 19, 4756 (2006).

Une façon de satisfaire à cette condition est d'utiliser la méthode des sources images, comme mentionné dans l'article ci-dessus, mais aussi comme expliqué dans la thèse d'Aurélie Laidevant, « Méthodes optiques résolues en temps pour la tomographie de fluorescence dans les milieux diffusants », Université Joseph Fourier, Grenoble, 5 octobre 2006, p.55-56:

- on connait la solution en milieu infini pour une source placée en r+. Une façon de satisfaire $\phi(\mathbf{r},t)$ =0 $\forall \mathbf{r} \in \partial\Omega_{extrapolée}$ est de placer une source virtuelle, de contribution négative, à une position r-, symétrique de r+ par rapport à la frontière extrapolée $\partial\Omega_{extrapolée}$.

Et tout ceci est valable également dans le domaine fréquentiel, et l'on a:

$$\widetilde{\Phi}^{1/2\infty}(\omega) = \widetilde{\Phi}^{\infty+}(\omega) - \widetilde{\Phi}^{\infty-}(\omega) = \frac{1}{4\pi c_n D}\left[\frac{e^{ik(\omega)r_+}}{r_+} - \frac{e^{ik(\omega)r_-}}{r_-}\right] = \frac{1}{4\pi c_n D}[G^{\infty}(k(\omega), r_+) - G^{\infty}(k(\omega), r_-)]$$

avec :

$$k^2(\omega) = -\frac{\mu_a}{D} + i\frac{\omega}{c_n D}$$

et :

$\tilde{\Phi}^{\infty+}$ : transformée de Fourier du débit de fluence émis par la source positive dans l'hypothèse d'un milieu infini

$\tilde{\Phi}^{\infty-}$ : transformée de Fourier du débit de fluence émis par la source négative dans l'hypothèse d'un milieu infini r+

= $|r_s^+ - r|$ distance à la source excitatrice positive, la source excitatrice positive étant la source excitatrice

r- = $|r_s^- - r|$: distance à la source excitatrice négative, cette source négative étant factice, et définie afin d'obtenir

$\phi(\mathbf{r},t)=0 \; \forall \mathbf{r} \in \partial\Omega_{extrapolée}$

$r_{s+}$ : coordonnées de la source excitatrice positive

$r_{s-}$ : coordonnées de la source excitatrice négative $G^{\infty}(k(\omega),r)$ : fonction de Green satisfaisant l'équation de diffusion en milieu infini. $G^{\infty}(k(\omega),r) = \dfrac{e^{ik(\omega)r}}{r}$

D'autre part, on a vu que $m_1 = <t> = i\dfrac{\partial\tilde{\Phi}(\omega)}{\partial\omega}\Big|\omega=0 \times \dfrac{1}{\tilde{\Phi}(\omega)|\omega=0}$

On en déduit, pour le milieu semi-infini :

$$<t>_{x,1/2\infty} = \frac{1}{2c_n\sqrt{\mu_{ax}D_x}}\frac{\dfrac{e^{ik(0)r_+} - e^{ik(0)r_-}}{e^{ik(0)r_+}} }{\dfrac{e^{ik(0)r_+}}{r_+} - \dfrac{e^{ik(0)r_-}}{r_-}} = \frac{1}{2c_n\sqrt{\mu_{ax}D_x}}\frac{e^{-\sqrt{\frac{\mu_{ax}}{D_x}}|\mathbf{r_{s+}}-\mathbf{r}|} - e^{-\sqrt{\frac{\mu_{ax}}{D_x}}|\mathbf{r_{s-}}-\mathbf{r}|}}{\dfrac{e^{-\sqrt{\frac{\mu_{ax}}{D_x}}|\mathbf{r_{s+}}-\mathbf{r}|}}{|\mathbf{r_{s+}}-\mathbf{r}|} - \dfrac{e^{-\sqrt{\frac{\mu_{ax}}{D_x}}|\mathbf{r_{s-}}-\mathbf{r}|}}{|\mathbf{r_{s-}}-\mathbf{r}|}} = \frac{f(|\mathbf{r_{s+}}-\mathbf{r}|,|\mathbf{r_{s-}}-\mathbf{r}|,k_x(0))}{2c_n\sqrt{\mu_{ax}D_x}}$$

$\Leftrightarrow$

$$<t>_{x,1/2\infty} = \frac{1}{2c_n\sqrt{\mu_a D}}\frac{r_+ G^{\infty}(k(0),r_+) - r_- G^{\infty}(k(0),r_-)}{G^{\infty}(k(0),r_+) - G^{\infty}(k(0),r_-)}$$

De même, on peut montrer que

$$<t>_{m,1/2\infty} = \frac{1}{2c_n\sqrt{\mu_{am}D_m}}\frac{\dfrac{e^{ik(0)r'_+} - e^{ik(0)r'_-}}{e^{ik(0)r'_+}}}{\dfrac{e^{ik(0)r'_+}}{r'_+} - \dfrac{e^{ik(0)r'_-}}{r'_-}} = \frac{1}{2c_n\sqrt{\mu_{am}D_m}}\frac{e^{-\sqrt{\frac{\mu_{am}}{D_m}}|\mathbf{r_{d+}}-\mathbf{r}|} - e^{-\sqrt{\frac{\mu_{am}}{D_m}}|\mathbf{r_{d-}}-\mathbf{r}|}}{\dfrac{e^{-\sqrt{\frac{\mu_{am}}{D_m}}|\mathbf{r_{d+}}-\mathbf{r}|}}{|\mathbf{r_{d+}}-\mathbf{r}|} - \dfrac{e^{-\sqrt{\frac{\mu_{am}}{D_m}}|\mathbf{r_{d-}}-\mathbf{r}|}}{|\mathbf{r_{d-}}-\mathbf{r}|}} = \frac{f(|\mathbf{r_{d+}}-\mathbf{r}|,|\mathbf{r_{d-}}-\mathbf{r}|,k_m(0))}{2c_n\sqrt{\mu_{am}D_m}}$$

$\Leftrightarrow$

Avec :

$r_d^+$ : position de la source positive, qui est dans ce cas la source fluorescente

$r_d^-$ : position de la source négative de la source fluorescente, source factice permettant d'obtenir $\phi_m(\mathbf{r},t)=0 \; \forall \mathbf{r} \in \partial\Omega_{extrapolée}$

r'+ = $|r_d^+ - r|$= distance à la source fluorescente positive

r'- = $|r_d^- r|$= distance à la source fluorescente négative

L'utilisation d'une source image en milieu semi-infini est également décrite dans l'article de M.S.Paterson et al. « time resolved reflectance and transmittance for the non invasive measurement of tissue optical properties », Applied Optics, vol.28, p.2331 - 2336, 1989. Ce document traite également d'une géométrie en forme de « tranche » (« slab »).

On peut montrer que pour le signal de fluorescence, on a toujours :

$$< t >_{1/2\infty} = < t >_{x,\,1/2\infty} + < t >_{m,\,1/2\infty} + \tau$$
$$= \frac{f\left(|\mathbf{r_{s+}} - \mathbf{r}|, |\mathbf{r_{s-}} - \mathbf{r}|, k_x(0)\right)}{v_x} + \frac{f\left(|\mathbf{r} - \mathbf{r_{d+}}|, |\mathbf{r} - \mathbf{r_{d-}}|, k_m(0)\right)}{v_m} + \tau$$

Ceci définit une fonction implicite et une surface dans l'espace en trois dimensions.

Là encore, et comme déjà expliqué ci dessus, pour trouver la position 3D du fluorophore, on effectue 3 mesures à trois positions de sources et 3 positions de détecteurs. Le fluorophore est donc à l'intersection de 3 surfaces définies dans l'espace par 3 équations différentes. Pour chaque couple de positions, on procède à l'établissement d'une TPSF ou d'un histogramme comme expliqué ci-dessus.

De préférence, on sélectionne des couples de positions définissant des directions sensiblement perpendiculaires les unes aux autres.

En pratique, les conditions que définit la géométrie en milieu semi-infini ne sont pas très différentes de celles du milieu infini, dès lors que l'on positionne la source et le détecteur (dans le cas de la figure 1A, les extrémités des fibres 10 et 12) loin de l'interface, à plus de 1 cm ou 2 cm de celle - ci (comme en figure 10A). Dans ce cas, en effet, tout se passe comme si le milieu était infini, du point de vue du fluorophore.

On peut vérifier la validité de l'équation

$$< t >_{1/2\infty} = < t >_{x,\,1/2\infty} + < t >_{m,\,1/2\infty} + \tau$$
$$= \frac{f\left(|\mathbf{r_{s+}} - \mathbf{r}|, |\mathbf{r_{s-}} - \mathbf{r}|, k_x(0)\right)}{v_x} + \frac{f\left(|\mathbf{r} - \mathbf{r_{d+}}|, |\mathbf{r} - \mathbf{r_{d-}}|, k_m(0)\right)}{v_m} + \tau$$

en se plaçant dans le cas d'une géométrie infinie. Il n'y a pas de source fictive et on a donc :

- $r_{s+} = r_s$,
- $r_- = |r_{s-} - r| - +\infty$,
- $r'_- = |r - r_{d-}| = +\infty$,
- $r_{d+} = r_d$

On retrouve alors l'expression correspondant au milieu infini.

On va traiter maintenant le cas d'un milieu à faces parallèles (géométrie en forme de « tranche » ou « slab »). Il est déjà question de ce type de géométrie dans l'article de M.S.Paterson et al. déjà cité ci-dessus.

Dans ce cas, on montre que, pour le signal d'excitation :

$$\widetilde{\Phi}_x^{\ slab}(\omega) = \sum_i \left[\widetilde{\Phi}_x^{\ \infty+}(r_{s+,i},\omega) - \widetilde{\Phi}_x^{\ \infty-}(r_{s-,i},\omega)\right] = \frac{1}{4\pi c_n D_x} \sum_i \left[\frac{e^{ik(\omega)r_{i+}}}{r_{i+}} - \frac{e^{ik(\omega)r_{i-}}}{r_{i-}}\right]$$

La sommation dans cette expression résulte du fait que l'on est en présence de deux plans parallèles qui limitent le milieu. Il y a donc contribution de deux sources virtuelles négatives, mais chacun des deux plans multiplie les effets de la source virtuelle négative associée à l'autre plan. Il faut donc ajouter la contribution de ces différents effets, sous la forme d'une somme à l'infini indicée par i.

De façon analogue, pour le signal d'émission :

$$\widetilde{\Phi}_m^{\ slab}(\omega) = \sum_i \left[\widetilde{\Phi}_m^{\ \infty+}(r'_{+,i},\omega) - \widetilde{\Phi}_m^{\ \infty-}(r_{-,i},\omega)\right] = \frac{1}{4\pi c_n D_m} \sum_i \left[\frac{e^{ik(\omega)r'+_i}}{r'_{+i}} - \frac{e^{ik(\omega)r'-_i}}{r'_{-i}}\right]$$

On appelle $<t>_{slab}$ le moment normé d'ordre 1 de $\Phi^{slab}(t)$, pouvant être obtenu par la transformée de Fourier

$$\widetilde{\Phi}slab\,(\omega) \text{ et } m_1 = < t > = i \frac{\partial \widetilde{\Phi}slab(\omega)}{\partial \omega}\bigg|\omega = 0 \times \frac{1}{\widetilde{\Phi}slab(\omega)\big|\omega = 0} \quad :$$

$$< t >_x^{slab} = \frac{1}{2c_n\sqrt{\mu_{ax}D_x}} \sum_i \frac{r_{+,i}\,G^\infty(k(0),r_{+,i}) - r_{-,i}\,G^\infty(k(0),r_{-,i})}{G^\infty(k(0),r_{+,i}) - G^\infty(k(0),r_{-,i})} = \sum_i \frac{f(|\mathbf{r}_{s+,i}-\mathbf{r}|,|\mathbf{r}_{s-,i}-\mathbf{r}|,k(0))}{2c_n\sqrt{\mu_{ax}D_x}}$$

et

$$< t >_m^{slab} = \frac{1}{2c_n\sqrt{\mu_{am}D_m}} \sum_i \frac{r'_{+,i}\,G^\infty(k(0),r'_{+,i}) - r'_{s-,i}\,G^\infty(k(0),r'_{-,i})}{G^\infty(k(0),r'_{+,i}) - G^\infty(k(0),r'_{-,i})} = \sum_i \frac{f(|\mathbf{r}_{d+,i}-\mathbf{r}|,|\mathbf{r}_{d-,i}-\mathbf{r}|,k(0))}{2c_n\sqrt{\mu_{am}D_m}}$$

Or,

$$< t >^{slab}_{1/2\infty} = < t >^{slab}_{x,1/2\infty} + < t >^{slab}_{m,1/2\infty} + \tau$$

Soit :

$$< t >_{slab} = \sum_i \left[ \frac{f(|\mathbf{r}_{\mathbf{s}i+}-\mathbf{r}|,|\mathbf{r}_{\mathbf{s}i-}-\mathbf{r}|,k_x(0))}{\nu_x} + \frac{f(|\mathbf{r}-\mathbf{r}_{\mathbf{d}i+}|,|\mathbf{r}-\mathbf{r}_{\mathbf{d}i-}|,k_m(0))}{\nu_m} \right] + \tau$$

Dans la pratique cependant, pour des milieux d'épaisseur suffisante, la somme converge assez rapidement, par exemple lorsque i est égal à 10 ou voisin de 10, par exemple i = 8, 9, 11 ou 12.

La surface décrite est encore une surface 3D. Donc, là encore, on localise un fluorophore par recherche des intersections entre 3 surfaces, chacune obtenue pour un couple de point (position source, position mesure) différent.

Pour trouver la position 3D du fluorophore, on effectue 3 mesures avec trois positions différentes de sources et 3 positions différentes de détecteurs, comme déjà expliqué ci-dessus.

On va traiter maintenant le cas d'un milieu de forme quelconque : ce milieu est limité par une enveloppe, mais la forme de celle-ci est a priori indéterminée. La méthode la plus générale pour traiter ce cas est la suivante.

On procède d'abord à un maillage du milieu.

Les équations sont ensuite résolues de manière numérique (par exemple par la méthode des volumes finis, ou la méthode des éléments finis, etc...).

On peut exprimer le signal de fluorescence de la manière suivante :

$$\Phi_m(\mathbf{r}_s,\mathbf{r}_d,\mathbf{r},t) \propto \phi_x(\mathbf{r}_s,\mathbf{r},t) * F(t) * \phi_m(\mathbf{r},\mathbf{r}_d,t)\,,$$

où :

$$F(t) = \frac{e^{-t/\tau}}{\tau}\,,$$

$$\frac{1}{c_n}\frac{\partial\phi_x(\mathbf{r},t)}{\partial t} + \vec{\nabla}.(-D_x(\mathbf{r})\nabla\phi_x(\mathbf{r},t)) + \mu_{ax}(\mathbf{r})\phi_x(\mathbf{r},t) = \delta(\mathbf{r}_s-\mathbf{r})\delta(t)\,,$$

avec la condition aux limites (3) ci-dessus.

Et :

$$\frac{1}{c_n}\frac{\partial\phi_m(\mathbf{r},t)}{\partial t} + \vec{\nabla}.(-D_m(\mathbf{r})\nabla\phi_m(\mathbf{r},t)) + \mu_{am}(\mathbf{r})\phi_m(\mathbf{r},t) = \delta(\mathbf{r}_d-\mathbf{r})\delta(t)\,,$$

avec la condition aux limites (4) ci-dessus.

On obtient donc ainsi, en tout point $\mathbf{r}$ la valeur des fonctions $\phi_x(\mathbf{r}_s,\mathbf{r},t)$ et $\phi_m(\mathbf{r},\mathbf{r}_d,t)$, dont on peut calculer les valeurs des moments, également en tout point **r**, en appliquant la relation (11) ci-dessus.

On a donc la relation :

$$\boxed{\begin{aligned} < t >_{signal\ mesuré} &=< t >_x + < t >_m + < t >_{déclin\ fluo} \\ &= f'(|\mathbf{r_s} - \mathbf{r}|) + f'(|\mathbf{r} - \mathbf{r_d}|) + \tau \end{aligned}}\quad,$$

où les fonctions f' sont donc calculées numériquement. On cherche ensuite les points **r** tels que cette relation soit vraie.

On peut inclure les paramètres D et $\mu$ dans la fonction f' et ainsi obtenir l'expression :

$$\texttt{<t>signal mesuré = f}_x\texttt{'(Dx, }\mu\texttt{ax, abs(rs-r)) +}$$
$$\texttt{f}_m\texttt{'(Dm, }\mu\texttt{am, }|\texttt{r-rd}|\texttt{) + }\tau\texttt{,}$$

avec :

$f_x'(Dx, \mu ax, |rs-r|)$ : solution de l'équation d'excitation
$f_m'(Dm, \mu am, |r-rd|)$ : solution de l'équation d'émission.

Par résolution numérique, on trouve des points r qui satisfont à cette équation et on peut définir une surface passant par tous ces points, par exemple par interpolation d'un point à l'autre. Cette surface n'est pas nécessairement un ellipsoïde.

Enfin, on peut traiter le cas où le milieu n'est pas homogène du point de vue de ses propriétés optiques, qui deviennent alors dépendantes de r. Dans ce cas, comme dans le cas d'un milieu homogène de forme quelconque, on procède à la résolution numérique de 3 équations, et on identifie le couple de points satisfaisant aux trois équations.

On détermine alors numériquement l'expression de $\tilde{\Phi}(\omega)$ pour ce milieu, satisfaisant l'équation de diffusion écrite dans le domaine fréquentiel :

$$\vec{\nabla}.(-D(\mathbf{r})\nabla\tilde{\Phi}(\mathbf{r},\omega)) + \left(\mu_a(\mathbf{r}) - \frac{i\omega}{c_n}\right)\tilde{\Phi}(\mathbf{r},\omega) = S(\mathbf{r}_s)$$

Et on en déduit ensuite $< t >= i\dfrac{\partial\tilde{\Phi}(\omega)}{\partial\omega}\Big|\omega = 0 \times \dfrac{1}{\tilde{\Phi}(\omega)|\omega = 0}$

Quelle que soit la géométrie envisagée, il est possible de s'affranchir de la dépendance par rapport à la durée de vie $\tau$, en effectuant une 4ème mesure avec un 4ème couple de positions (position de la source, position du détecteur) différent de chacun des 3 précédents. Pour de couple supplémentaire de positions (source, détecteur), on procède à l'établissement d'une TPSF ou d'un histogramme comme expliqué ci-dessus. On obtient alors l'équation d'une 4ème surface.

Comme indiqué ci-dessus dans chacun des cas traités on obtient en fait un calcul du temps moyen à partir de chaque signal de fluorescence.

Il est décrit dans le document EP 1 884 765 que l'on peut réaliser le calcul d'une variable indépendante de $\tau$ en effectuant la différence entre le temps moyen calculé pour chaque signal de fluorescence et le temps moyen calculé pour un signal de fluorescence particulier, par exemple le signal de fluorescence particulier est celui ayant un temps moyen minimal ou ayant un temps moyen calculé minimal.

Parmi les 4 mesures effectuées pour les 4 couples de positions, on pourra sélectionner celle qui correspond à un temps moyen mesuré minimal. L'équation de la surface qui correspond à cette mesure minimale sera soustraite de chacune des 3 autres équations.

On obtiendra alors 3 nouvelles équations de 3 nouvelles surfaces, qui peuvent ne pas être des ellipsoïdes, mais à l'intersection desquelles sera localisé le fluorophore, ou bien le fluorophore sera localisé dans un volume qui contient cette intersection.

Quelle que soit la géométrie envisagée, le calcul de l'intersection de 3 ellipsoïdes, ou 3 surfaces, définis, ou définies, dans l'espace par 3 équations différentes est effectué à l'aide d'un traitement numérique mis en oeuvre par un calculateur, par exemple les moyens 24 des figures 1A et 1B. Ce traitement conduit à une localisation de l'intersection des 3 surfaces avec une certaine précision. Autrement dit, la résolution du système d'équations que constituent les définitions des 3 surfaces est effectuée avec une certaine précision, ce qui donnera une solution approchée : le fluorophore n'est alors pas localisé exactement à l'intersection de 3 surfaces, mais au voisinage de cette intersection.

Après avoir trouvé une solution avec une première précision, on peut effectuer de nouveau le calcul pour trouver une autre solution avec une deuxième précision, différente de la première.

Chaque surface peut elle-même être définie avec une certaine précision. Ceci définit non plus une surface au sens strict, mais un volume qui s'appuie sur ladite surface. Dans le cas d'un ellipsoïde, il peut s'agir d'une couronne ellipsoïdale. Ce volume est délimité par deux surfaces, sensiblement parallèles à la surface définie strictement par l'équation correspondante et proches de celle-ci la proximité étant définie par la précision associée à la surface, qui devra être de préférence inférieure à 25%, et encore de préférence inférieure à ± 10% .Autrement dit, on cherche alors non pas l'intersection de 3 surfaces, mais de 3 volumes. Il en résulte non pas un point unique, mais l'identification d'un volume, en général suffisamment petit pour être compatible avec la localisation avec une certaine incertitude; ce volume contient l'intersection des 3 surfaces telles que définies strictement par les 3 équations initiales.

D'une manière générale, on peut distinguer différents types d'analyse.

Il y a d'abord le cas d'une analyse invasive pour lequel source et détecteur sont placés à l'intérieur du milieu diffusant. Si la profondeur de la source et du détecteur par rapport à la frontière du milieu est suffisante, on considèrera qu'on se trouve dans les conditions d'un milieu infini (cas de la figure 10A).

Un premier cas d'analyse non invasive est celui pour lequel source et détecteur sont placés au contact d'une frontière du milieu diffusant (cas de la figure 10B).

Un deuxième cas d'analyse non invasive, est celui pour lequel source et détecteur ne sont pas en contact physique avec une frontière 21 du milieu diffusant 20, mais sont en contact optique avec le milieu. Dans ce cas, la source est par exemple un laser 8 focalisé à la surface 21 du milieu et le détecteur est un photodétecteur 4 en contact optique avec la surface du milieu (cas de la figure 10C).

Le premier cas non invasif peut-être combiné au second cas non invasif, la source 8 étant située à distance (comme le laser 8 de la figure 10D) et la détection s'effectuant au contact, par exemple à l'aide d'une fibre optique 12 qui amène le rayonnement à détecter vers le détecteur 4 (cas de la figure 10D). Par ailleurs, l'excitation peut s'effectuer au contact, par exemple au moyen de fibres optiques. Généralement, en mode non invasif, on ne considère pas qu'on se trouve dans une géométrie infinie, et on choisit alors un autre type de géométrie:

- la géométrie semi-infinie,
- ou la géométrie de type tranche,
- ou la géométrie de forme quelconque.

L'intérêt de se rapprocher de la géométrie infinie, semi-infinie ou de type tranche est de permettre d'utiliser une relation analytique reliant une grandeur mesurée (par exemple le temps moyen d'arrivée des photons) aux distances respectives source-fluorophore (ou agrégation ou accumulation locale de fluorophore) et détecteur -flurophore (ou agrégation ou accumulation locale de fluorophore). Ainsi, pour 3 acquisitions différentes, c'est-à-dire réalisée avec 3 couples différents (source - détecteur), le problème revient à déterminer les solutions satisfaisant à trois équations analytiques, ce qui peut-être entrepris rapidement à l'aide de moyens de calculs actuels. En revanche, comme décrit ci-dessous, le fait de se placer en géométrie quelconque revient à résoudre le problème de façon numérique, au détriment du temps de calcul.

Dans les différents cas expliqués ci-dessus en liaison avec les figures 10A-10D, la référence 20 désigne un milieu étudié, dont la limite est la paroi 21. Ce milieu 20 peut être par exemple un organe d'un animal ou d'un être humain, par exemple le cerveau, ou un sein (à titre d'exemples de fluorophores pour ces différents milieux, on peut citer le vert d'indocyanine, ou la fluorescéine).

Sur ces figures, le point F désigne un fluorophore ou une agrégation de fluorophores. Mais on rappelle qu'il peut s'agir aussi d'un absorbeur (ou encore d'un fluorophore ré-émettant à la même énergie que celle d'excitation, et avec une durée $\tau = 0$) ou d'une agrégation d'absorbeurs. Dans ce cas, le milieu 20 peut être encore un organe, la zone F identifiant une tumeur cancéreuse, visibles par le simple fait qu'elle présente un coefficient d'atténuation plus important que les tissus sains environnants.

Des exemples vont maintenant être décrits.

**Exemples :**

**- Exemple I-**

Cet exemple est un calcul en deux dimensions. Il ne concerne donc pas une mesure réelle, mais illustre la méthode dans un cas théorique, dans un plan.

Le milieu est supposé infini, à 2 dimensions, avec les propriétés optiques suivantes.

Le coefficient de diffusion réduit est $\mu'_s$ = 10 cm$^{-1}$ ce qui conduit à : D=1/3$\mu'_s$, (cette définition de D est la plus répandue, mais il existe d'autres définitions, par exemple D=1/3($\mu'_s$+ $\mu_a$), ce qui ne change rien si $\mu_a$ <<$\mu'_s$ qui définit en général le cadre de validité de l'approximation de la diffusion).

Le coefficient d'absorption est $\mu_a$ = 0,1cm$^{-1}$.

On suppose en outre que les propriétés optiques sont les mêmes aux deux longueurs d'ondes, d'indice de réfraction n=1.0, ce qui donne une vitesse de propagation dans le milieu $c_n$ = 3.10$^8$/n.

Il en résulte une vitesse apparente :

$$v_{app} = 2\frac{c}{n}\sqrt{\mu_a D} = 3.4469 \times 10^7 \text{ m.s}^{-1}.$$

Le fluorophore choisi a un temps de vie $\tau$=1.5 ns, ordre de grandeur classique des fluorophores que l'on utilise en imagerie moléculaire optique.

On suppose que l'on réalise :

- une première mesure $(<t>_\infty$-$\tau)\times v_{app}$ = 4.7000 cm, avec la source positionnée en surface du milieu en (0.0, 0.0) et un détecteur positionné en (1.0 cm,0.0). Cela signifie que le fluorophore est placé sur la demi-ellipse d'équation:

$$\xi_1 : \sqrt{(x-x_{s1})^2 + (y-y_{s1})^2} + \sqrt{(x-x_{d1})^2 + (y-y_{d1})^2} = v_{app} \times (\underbrace{\langle t \rangle_{mes1}}_{moment\,d'ordre\,1\,mesuré=m1} - \tau) = 4.7$$

- une seconde mesure $(<t>_\infty$-$\tau)\times v_{app}$ = 5.1000cm, avec la source positionnée en surface du milieu en (0.5 cm, 0.0) et un détecteur en (1.5 cm,0.0). Cela signifie que le fluorophore est placé sur la demi-ellipse d'équation

$$\xi_2 : \sqrt{(x-x_{s2})^2 + (y-y_{s2})^2} + \sqrt{(x-x_{d2})^2 + (y-y_{d2})^2} = v_{app} \times (\underbrace{\langle t \rangle_{mes2}}_{moment\,d'ordre\,1\,mesuré=m1} - \tau) = 5.1$$

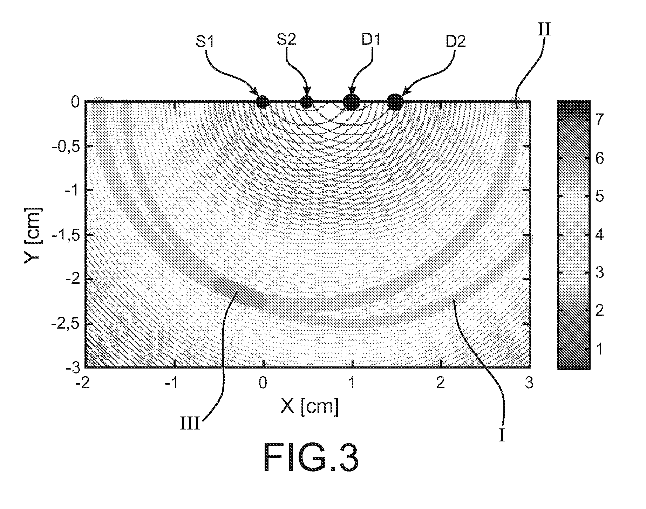

La figure 3 représente l'intersection (zone III) des deux ellipses I et II ainsi définies, cette intersection donne la position du fluorophore. En deux dimensions, le procédé selon l'invention donne donc des résultats satisfaisants. Des exemples en 3 dimensions vont maintenant être donnés.

Exemple **II-**

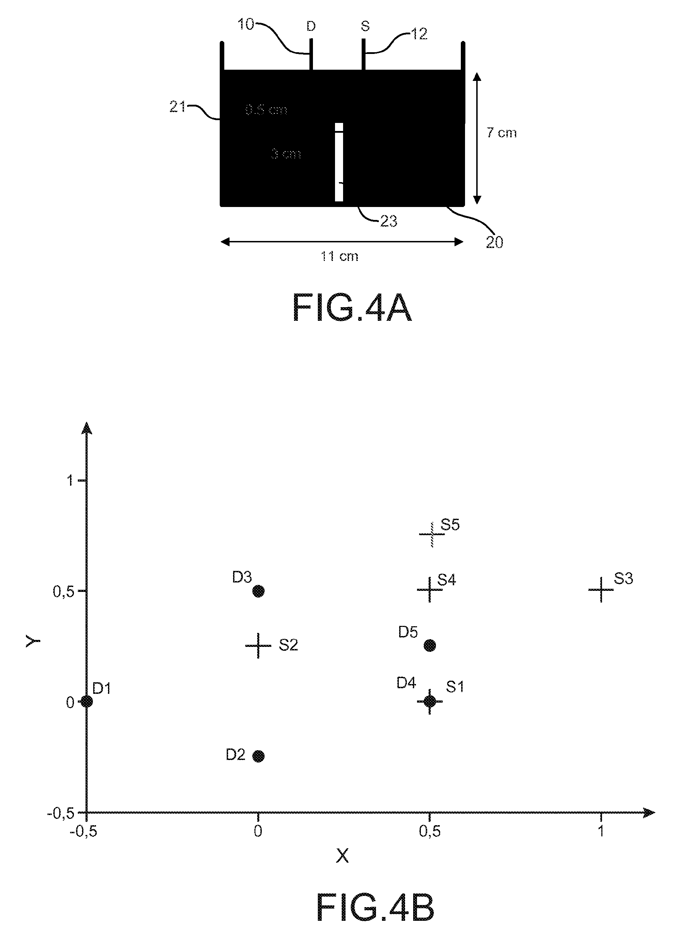

Le montage expérimental (figure 4A) est sensiblement celui de la figure 1A avec une diode laser 8 de longueur d'onde d'émission à environ 635 nm (diode laser de H&B) en tant que source d'excitation et un photomultiplicateur 4 couplé à une carte TCSPC.

La cuve 21 est remplie d'un milieu diffusant liquide 20 composé d'eau et de billes d'Acronal® (résine acrylique, BASF), ce qui conduit aux paramètres optiques suivants:

- $\mu a$ = 0.01 cm$^{-1}$,
- $\mu's$ = 8.7 cm$^{-1}$

On considère que les propriétés optiques sont identiques à la longueur d'onde d'excitation et à celle d'émission, du fait de la proximité des longueurs d'onde d'excitation et de diffusion, suffisante pour considérer ces coefficients constants sur cette plage de longueur d'onde.

Le milieu fluorescent considéré est du Cy5 de concentration 10$\mu$Mol. La durée de vie $\tau$ est de 0,96 ns, et l'indice de réfraction $n_0$ du milieu est de 1,33.

La figure 4A représente de manière plus détaillée le positionnement de l'échantillon dans la cuve 21. Celui-ci est placé dans un tube capillaire 23 en verre, de diamètre interne 1 mm, sur une hauteur d'environ 2 mm.

On retrouve également sur cette figure la portion des fibres 10 et 12 qui sont plongées dans le milieu examiné. L'extrémité de chaque fibre est située à 3,5 cm sous le niveau du liquide, ce qui suffit à assurer la condition de milieu infini. La puissance mesurée en sortie de fibre d'excitation 10 est de 100 $\mu$W.

Sont également représentées sur cette figure les positions respectives de l'échantillon et des extrémités de fibres.

Du point de vue de la modélisation, la source n'étant pas ponctuelle, tout se passe comme si elle devenait ponctuelle à une distance d'un libre parcours moyen de transport soit à une distance de $1/\mu$'s (ce qui est bien le cas dans la configuration envisagée, puisque la distance entre les extrémités de fibres et l'échantillon est de 0,5 cm, tandis que $1/\mu$'s < 0,12 cm < 0,5 cm).

On procède à 5 mesures, avec des positions de sources et de détecteurs indiquées ci-dessous.

On a sélectionné les 5 positions suivantes Si (i = 1,...5) de la source (en fait : l'extrémité de la fibre 10 ; toutes les coordonnées sont en cm):

- S1 : $r_{s1}$=[0.5, 0, -3.5],
- S2 : $r_{s2}$=[0, 0.25, -3.5],
- S3 : $r_{s3}$= [1, 0.5, -3.5],
- S4 : $r_{s4}$= [0.5, 0.5, -3.5];
- S5 : $r_{s5}$= [0.5, 0.75, -3.5];

Et les 5 positions suivantes Di (i = 1,...5) du détecteur (en fait : l'extrémité de la fibre 12;toutes coordonnées également en cm):

- D1: $r_{d1}$=[-0.5, 0, -3.5],
- D2: $r_{d2}$= [0, -0.25, -3.5],
- D3: $r_{d3}$= [0, 0.5, -3.5];
- D4: $r_{d4}$= [0.5, 0, -3.5] ;
- D5: $r_{d5}$= [0.5, 0.25, -3.5]

Pour la position Si de la source, le détecteur est en position Di.

La figure 4B donne chacune des positions Si et Di (i = 1,...5) dans le plan X, Y (Z est fixé à -3,5 cm). Les distances di calculées entre Si et Di sont : d1=2,27 cm ; d2=2,01 cm ; d3=2,73 cm ; d4=2,39 cm ; d5=2,53cm.

La figure 4C donne la courbe de fluorescence obtenue pour chaque couple de positions (Si, Di). Les courbes ont été préalablement traitées comme expliqué dans A.Laidevant et al., Applied Optics, 45, 19, 4756 (2006).

Les valeurs mesurées de $v_{app}$ ($<t>_{mes1}$) sont les suivantes (on rappelle que $<t>_{mes1}$ est le moment d'ordre 1 mesuré):

- Pour (S1, D1): 2, 2700 cm ;
- Pour (S2, D2): 2,0059 cm ;
- Pour (S3, D3): 2,7284 cm ;
- Pour (S4, D4): 2,3931 cm ;
- Pour (S5, D5): 2,5258 cm.

Partant des mesures effectuées pour 3 des 5 couples de points ((S1, D1), (S3, D3) et (S4, D4)), on a calculé trois surfaces tridimensionnelles, en fait trois ellipsoïdes, conformément à l'enseignement de l'invention.

L'intersection de ces trois surfaces a également été calculée.

Les 3 surfaces ont été élaborées en considérant un maillage :

- En x : de 50 points entre x = -0,5 cm et x=2 cm;
- En y : de 30 points entre y = -0,5 cm et y=1 cm;
- En z : de 50 points entre z = -6 cm et z=-3,5 cm.

Le fluorophore a été localisé en position $(x_1,y_1,z_1)$ suivante :

- $-0,041 < x_1 < 0,062$;
- $0,12 < y_1 < 0,17$
- $-0,91 < z_1 < -3,81$.

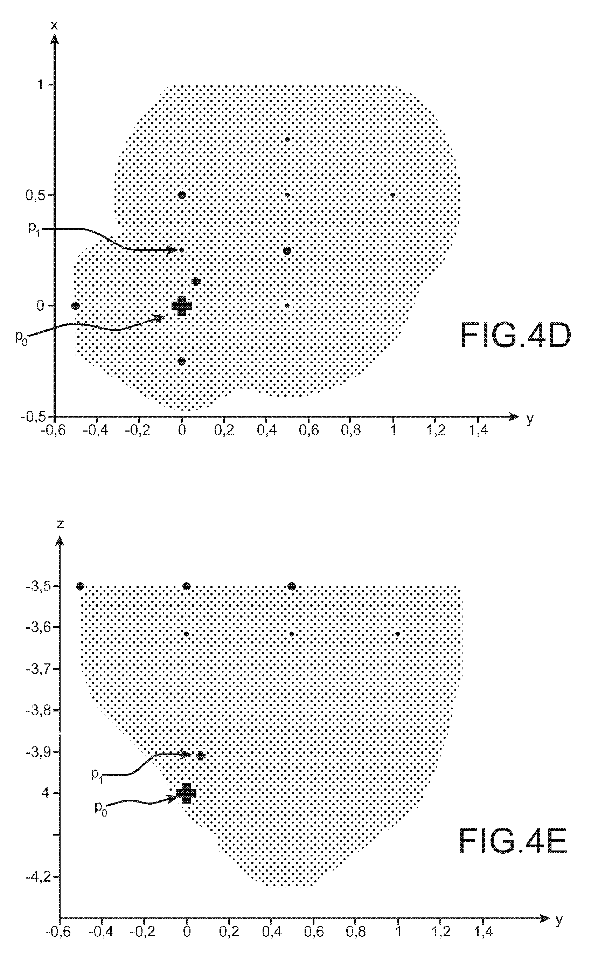

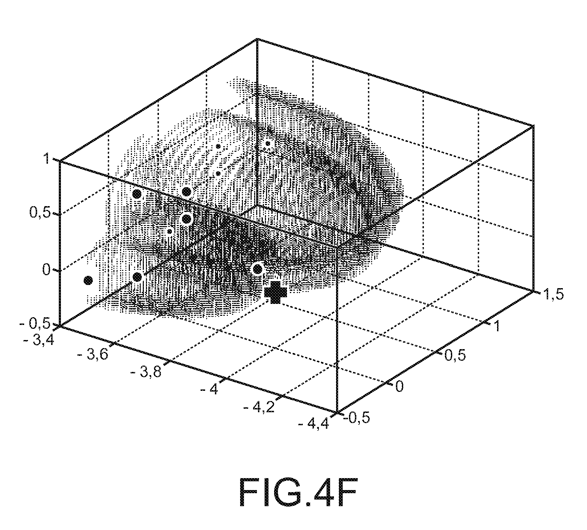

On rappelle que la position réelle est (0,0, -4). Les figures 4D et 4E représentent dans chacun des plans XY et ZY (donc respectivement en vue de dessus et en vue de côté de la cuve 21) la position $p_1$ mesurée et la position $P_0$ calculée, avec un léger décalage entre ces deux positions. La figure 4F représente ces deux positions en trois dimensions, avec, également, un léger décalage entre ces deux positions. Malgré ce décalage, on constate que l'information obtenue par le calcul est tout à fait satisfaisante pour bon nombre de cas, par exemple pour une localisation approchée dans un organe du corps humain.

Le temps nécessaire pour ces calculs, pour le maillage choisi (avec le maillage retenu) avec un logiciel « Matlab », sur processeur Intel Core2, à 2.13GHz, avec 1Go de RAM est inférieur à 3 secondes:

- durée du calcul des distances di pour les trois couples (Si, Di) sélectionnés: 2,6 s
- durée du calcul des équations des 3 ellipsoïdes ($\xi$1, $\xi$2, et $\xi$3): 0,12 s
- durée du calcul de la détermination de l'intersection : 0,04 s.

La durée totale de calcul avec un matériel informatique standard est tout à fait compatible avec une mesure sur ou dans un organe du corps humain, par exemple lors d'une intervention chirurgicale ou dans une unité d'analyse.

Le calcul a ensuite été refait, en prenant en compte l'ensemble des 5 mesures : on a donc calculé l'intersection des 5 surfaces.

Le fluorophore a alors été localisé en position $(x_2, y_2, z_2) = (0,06; 0,12; 3,91)$. Cette nouvelle position est assez proche de la précédente : la localisation effectuée avec seulement trois surfaces est donc suffisante.

**Exemple III-**

Cet exemple est en trois dimensions, et en milieu semi-infini.

On considère 3 couples de positions de la source et du détecteur $(r_s, r_d)$.

Par exemple, on a les 3 positions suivantes de la source (toutes les coordonnées sont en cm): $r_{s1}$=[0.0, 0, 0], $r_{s2}$= [0.5, 0, 0], $r_{s3}$= [0.75, -1, 0];

Et les 3 positions suivantes du détecteur (toutes coordonnées également en cm):

$r_{d1}$=[1.0, 0, 0], $r_{d2}$= [1.5, 0, 0], $r_{d3}$= [0.75, 0, 5, 0]

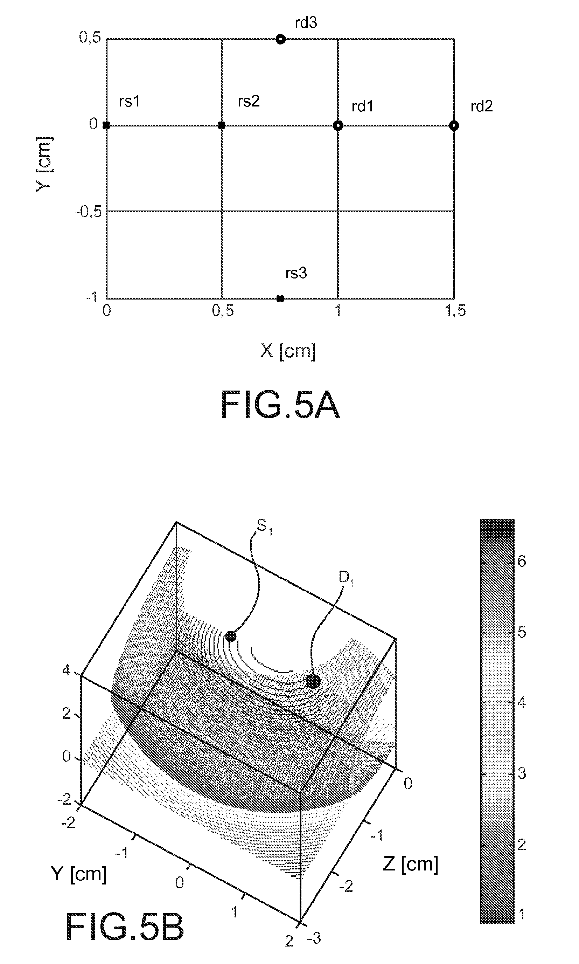

Ces 3 couples sont représentés en figure 5A.

Les propriétés optiques sont les mêmes que dans l'exemple précédent.

On suppose que l'on réalise :

- une première mesure $(<t>_\infty - \tau) \times v_{app}$ = 4.6000 cm, pour le couple de positions $(r_{s1}, r_{d1})$,
- une deuxième mesure $(<t>_\infty - \tau) \times v_{app}$ = 5.6000 cm, pour le couple de positions $(r_{s2}, r_{d2})$,
- une troisième mesure $(<t>_\infty - \tau) \times v_{app}$ = 4.6000 cm, pour le couple de positions $(r_{s3}, r_{d3})$.

Alors la position du fluorophore appartient à l'intersection des ellipsoïdes, qui ont pour équations suivantes:

$$\xi_2 : f(|\mathbf{r_{s2+}} - \mathbf{r}|, |\mathbf{r_{s2-}} - \mathbf{r}|, k(0)) + f(|\mathbf{r} - \mathbf{r_{d2+}}|, |\mathbf{r} - \mathbf{r_{d2-}}|, k(0)) = v_{app} \times ( \underbrace{\langle t \rangle_{mes1}}_{moment\ d'ordre\ 1\ mesuré=m1} - \tau ) = 4.6$$

$$\xi_3 : f(|\mathbf{r_{s3+}} - \mathbf{r}|, |\mathbf{r_{s3-}} - \mathbf{r}|, k(0)) + f(|\mathbf{r} - \mathbf{r_{d3+}}|, |\mathbf{r} - \mathbf{r_{d3-}}|, k(0)) = v_{app} \times ( \underbrace{\langle t \rangle_{mes1}}_{moment\ d'ordre\ 1\ mesuré=m1} - \tau ) = 5$$

$$\xi_1 : f(|\mathbf{r_{s1+}} - \mathbf{r}|, |\mathbf{r_{s1-}} - \mathbf{r}|, k(0)) + f(|\mathbf{r} - \mathbf{r_{d1+}}|, |\mathbf{r} - \mathbf{r_{d1-}}|, k(0)) = v_{app} \times ( \underbrace{\langle t \rangle_{mes1}}_{moment\ d'ordre\ 1\ mesuré=m1} - \tau ) = 4.6$$

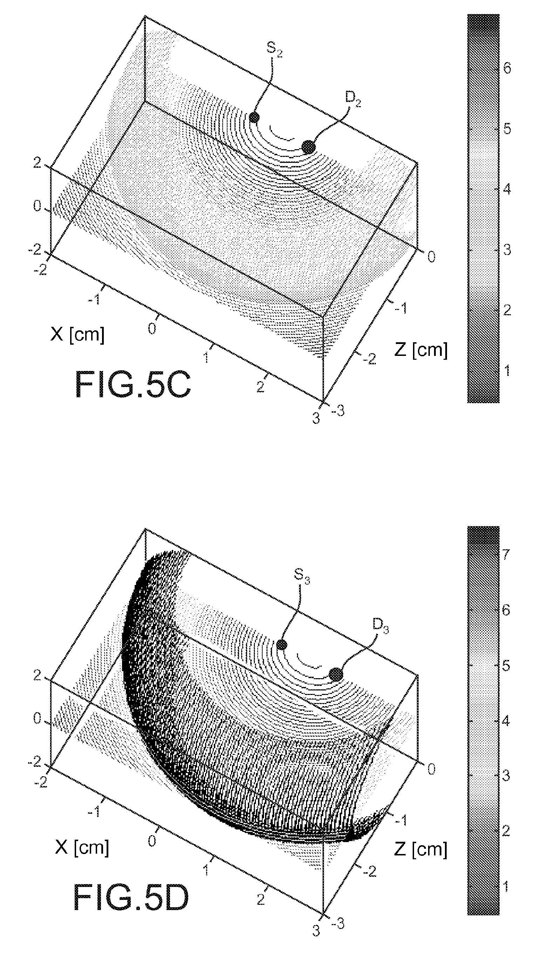

Sur chacune des figures 5B, 5C, 5D est représentée une partie de chacun des ellipsoïdes correspondants.

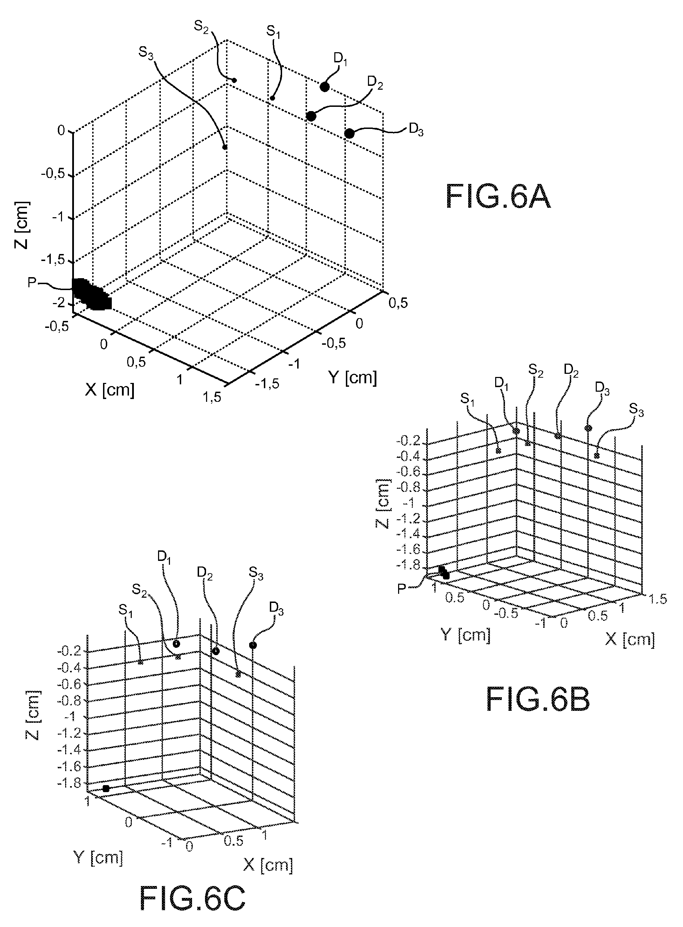

La zone d'intersection de ces 3 ellipsoïdes est identifiée par la zone I en figure 6A, sur laquelle sont en outre représentées les trois positions S1, S2, S3 de la source et les trois positions D1, D2, D3 du détecteur utilisées pour les trois mesures. La zone P est la zone de localisation du fluorophore.

Les figures ont été élaborées en considérant un maillage :

- En x : de 101 points entre x = -2 et x=3;
- En y : de 101 points entre x = -2 et x=2;
- En z : de 101 points entre x = -3 et x=0;

La solution de la figure 6A est une solution approchée, à 10% près.

Il est possible d'imposer une précision plus importante. Ainsi, en figures 6B et 6C, sont représentées des solutions (zone P) plus précises :

En figure 6B : solution à 2% près,
En figure 6C : solution à 1,4% près.

On voit que l'accroissement de précision apporte peu d'information supplémentaire pour une localisation approchée du fluorophore.

Il est intéressant d'évaluer le temps nécessaire pour ces calculs, avec un logiciel « Matlab », sur processeur Intel Core2, à 2.13GHz, avec 1Go de RAM:

- pour le calcul des moments pour les 3 couples source-détecteur en chaque point du maillage : 5.6835 s,
- pour le calcul des équations des 3 ellipsoïdes ($\xi 1$, $\xi 2$, et $\xi 3$): 2.086609 s,
- pour le calcul de la détermination de l'intersection : 2.253164 s.

Il en résulte une durée totale d'environ 10 s pour le maillage choisi.

Ce résultat illustre bien le caractère « temps réel » ou rapide du procédé.

Dans le cadre d'une application chirurgicale, un praticien peut réaliser une analyse selon l'invention alors même qu'il est en présence d'un patient. Une durée de 10 s, ou même une durée légèrement supérieure, est tout à fait compatible avec le maintien d'un patient sur une table d'opération ou dans une salle d'analyse.

La détermination de la position du fluorophore dépend de son temps de vie. Pour s'en affranchir, on peut considérer des mesures différentielles, comme expliqué dans la demande de brevet EP 1 884 765.

Par exemple :

Avec les configurations de sources-détecteurs ci-dessus, en milieu semi-infini, on a les ellipsoïdes suivants :

$$\xi_1 : f(|\mathbf{r_{s1+}} - \mathbf{r}|, |\mathbf{r_{s1-}} - \mathbf{r}|, k(0)) + f(|\mathbf{r} - \mathbf{r_{d1+}}|, |\mathbf{r} - \mathbf{r_{d1-}}|, k(0)) = v_{app} \times (\underbrace{\langle t \rangle_{mes1}}_{moment\,d'ordre\,1\,mesuré=m1} - \tau) = 4.7$$

$$\xi_2 : f(|\mathbf{r_{s2+}} - \mathbf{r}|, |\mathbf{r_{s2-}} - \mathbf{r}|, k(0)) + f(|\mathbf{r} - \mathbf{r_{d2+}}|, |\mathbf{r} - \mathbf{r_{d2-}}|, k(0)) = v_{app} \times (\underbrace{\langle t \rangle_{mes1}}_{moment\,d'ordre\,1\,mesuré=m1} - \tau) = 5$$

$$\xi_3 : f(|\mathbf{r_{s3+}} - \mathbf{r}|, |\mathbf{r_{s3-}} - \mathbf{r}|, k(0)) + f(|\mathbf{r} - \mathbf{r_{d3+}}|, |\mathbf{r} - \mathbf{r_{d3-}}|, k(0)) = v_{app} \times (\underbrace{\langle t \rangle_{mes1}}_{moment\,d'ordre\,1\,mesuré=m1} - \tau) = 4.6$$

Le 3ème correspond à une mesure dont le trajet optique est le plus court des trois. C'est cette mesure qui, comme expliqué dans la demande EP 1 884 765, peut être utilisée comme mesure de référence.

Si l'on considère les quantités

$$\xi_1 - \xi_3 = 0.1$$

et

$$\xi_2 - \xi_3 = 0.4 \quad ,$$

on obtient des relations indépendantes du temps de vie.

Ces quantités ne définissent plus des ellipsoïdes mais des surfaces plus complexes.

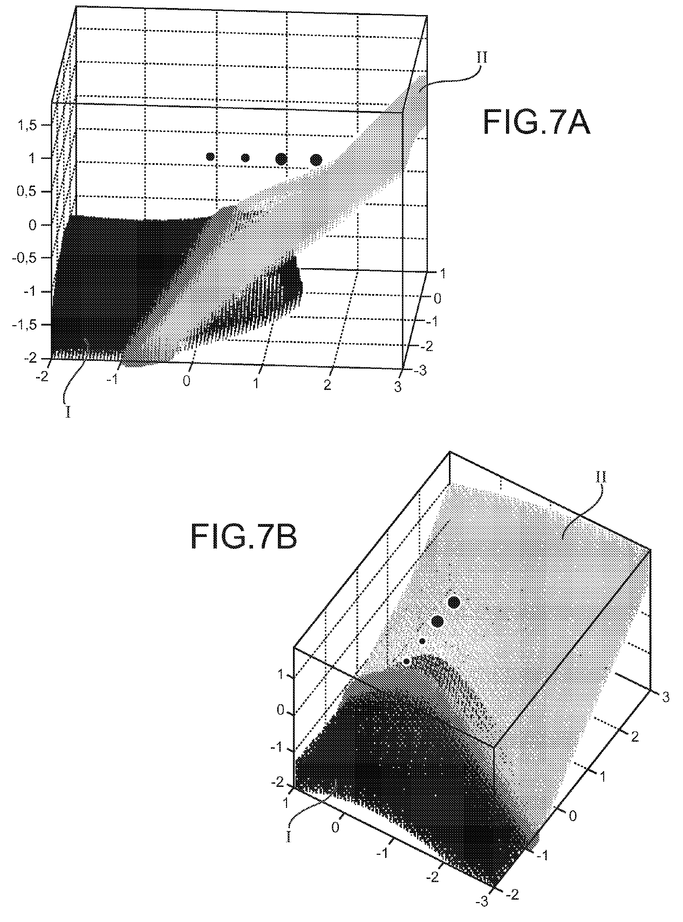

Les figures 7A et 7B représentent, suivant deux orientations différentes, l'intersection entre les deux surfaces 3D $\xi_1$-$\xi_3$ (zone I sur ces figures) et $\xi_2$-$\xi_3$ (zone II sur ces figures).

En 3D, une troisième surface est nécessaire pour identifier une localisation possible du fluorophore.

On considère donc un ensemble, non pas de 3 mesures, mais de 4 mesures.

Par exemple, on a les 4 positions suivantes de la source (toutes les coordonnées sont en cm): $r_{s1}$=[0.0, 0, 0], $r_{s2}$= [0.5, 0, 0], $r_{s3}$= [0.75, -1, 0]; $r_{s4}$= [-0.75 ; -1.5 ; 0];

Et les 4 positions suivantes du détecteur (toutes coordonnées également en cm) :

$r_{d1}$=[1.0, 0, 0], $r_{d2}$= [1.5, 0, 0], $r_{d3}$= [0.75, 0,5, 0] ; $r_{d4}$= [0.75, 1, 0].

On obtient donc, outre les 3 surfaces $\xi_1$, $\xi_2$, $\xi_3$ déjà définies, une surface $\xi_4$ (qui définit elle aussi un ellipsoïde) :

$$\xi_4 : f(|\mathbf{r_{s4+}} - \mathbf{r}|, |\mathbf{r_{s4-}} - \mathbf{r}|, k(0)) + f(|\mathbf{r} - \mathbf{r_{d4+}}|, |\mathbf{r} - \mathbf{r_{d4-}}|, k(0)) = v_{app} \times ( \underbrace{\langle t \rangle_{mes1}}_{moment\ d'ordre\ 1\ mesuré = m1} - \tau) = 5.0$$

Cette surface $\xi_4$ va elle aussi être normalisée. On effectue donc la différence $\xi_4$-$\xi_3$ qui définit elle-même une surface dont l'intersection avec les surfaces $\xi_1$-$\xi_3$ et $\xi_2$-$\xi_3$ va permettre de localiser le fluorophore.

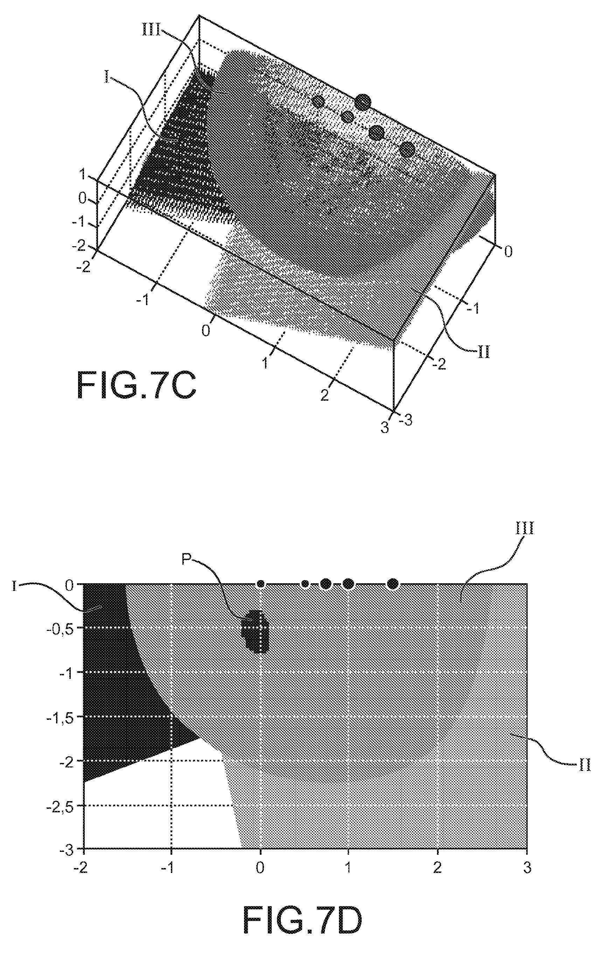

La figure 7C représente les 3 surfaces $\xi_1$-$\xi_3$, (zone I), $\xi_2$-$\xi_3$ (zone II) et $\xi_4$-$\xi_3$ (zone III), tandis que la figure 7D représente une coupe de l'ensemble dans un plan XZ.

La zone d'intersection des 3 surfaces, et donc la zone de localisation du fluorophore est identifiée par la zone P sur cette figure 7D.

Exemple **IV-**

Cet exemple est en trois dimensions, et en géométrie de type « slab ».

La tranche considérée est d'épaisseur L = 1, 5 cm.

On considère 3 couples de positions de la source et du détecteur ($r_s$, $r_d$).

Par exemple, on a les 3 positions suivantes de la source (toutes coordonnées sont en cm): $r_{s1}$=[0.0, 0, 0], $r_{s2}$= [0.5, 0, 0], $r_{s3}$= [0.75, -1, 0];

Et les 3 positions suivantes du détecteur (toutes coordonnées également en cm) :

$r_{d1}$=[1.0, 0, 0], $r_{d2}$= [1.5, 0, 0], $r_{d3}$= [0.75, 0, 5, 0]

Comme pour les exemples précédents, on obtient la définition de trois ellipsoïdes, pour les trois valeurs suivantes de $v_{app}$ (<t>$_{mes}$) : 1,84 ; 1,93 ; 1,81.

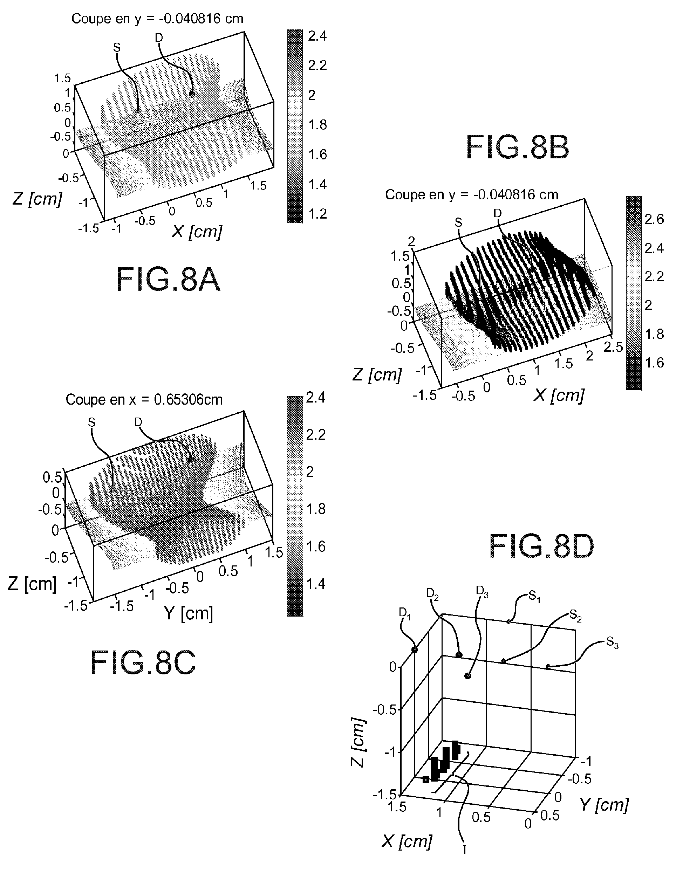

Sur chacun des figures 8A, 8B, 8C est représentée une partie de chacun des ellipsoïdes correspondants.

Sur ces figures, la tranche constituant le milieu est délimitée par les deux plans parallèles: Z = 0 et Z = - 1,5 cm.

La zone d'intersection de ces 3 ellipsoïdes est identifiée par la zone I en figure 8D, sur laquelle sont en outre représentées les trois positions de la source et les trois positions du détecteur, utilisées pour les trois mesures.

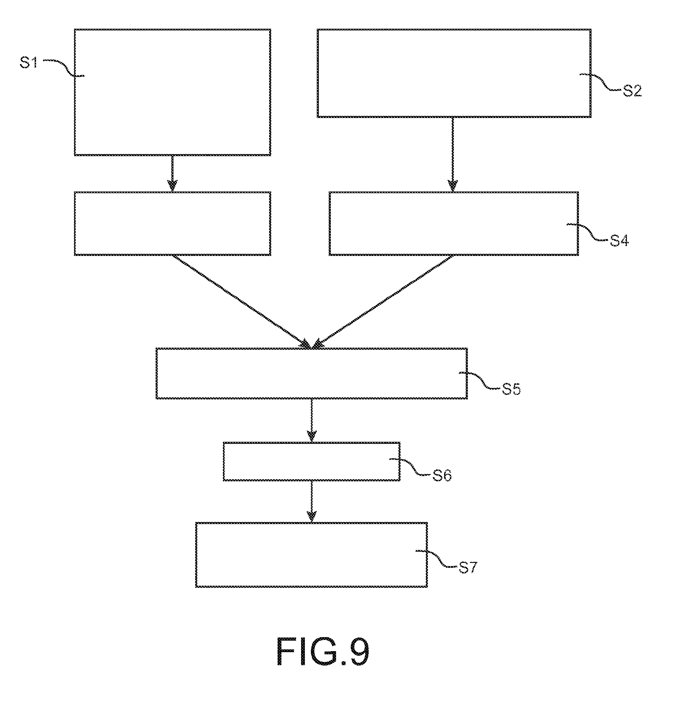

De manière générale, un exemple de procédé selon l'invention peut être mis en oeuvre selon le schéma représenté en figure 9.

Les étapes de cet exemple, et d'ailleurs de toute autre mise en oeuvre d'un procédé selon l'invention, peuvent être précédées d'une étape - non représentée - d'injection dans le milieu étudié d'au moins un fluorophore ou absorbeur destiné à venir se fixer sur une zone d'intérêt. En fait lorsqu'on injecte un fluorophore, on en injecte souvent plusieurs. On peut d'ailleurs assimiler une zone fluorescente à une agglomération de fluorophores. Il n'y a généralement pas qu'une seule molécule fluorescente, mais une concentration locale de molécules fluorescentes. De la même façon, lorsqu'on travaille en absorption, il n'y a pas qu'une seule molécule absorbante, mais une concentration de molécules absorbantes. Cette concentration peut être considérée comme ponctuelle si la distance la séparant du détecteur est suffisamment grande devant ses dimensions. Typiquement, une concentration de rayon r est considérée comme ponctuelle, à une distance d'observation d donnée, si d > 10 r. Certains absorbeurs peuvent déjà être dans le milieu, par exemple lorsqu'il s'agit d'une zone présentant une absorption différente de son environnement.

On positionne ensuite les moyens d'émission de rayonnement et de détection par rapport au milieu. L'une des configurations déjà décrites peut être sélectionnée. En particulier on peut réaliser un examen invasif u non invasif.

Au cours d'une première étape S1 de ce procédé, les données, ou paramètres, d'entrée sont fournis: il s'agit des propriétés optiques du milieu diffusant, aux deux longueurs d'onde de d'excitation et d'émission (dans certains cas, à ces deux longueurs d'onde, les propriétés optiques sont identiques), la géométrie du milieu, les positions des sources et du détecteur (au sens déjà expliqué ci-dessus), et le choix du maillage pour le calcul des surfaces. Au cours d'une deuxième étape (S2), les fichiers de mesures (TPSF) pour chaque couple source-détecteur sont lus. Un prétraitement des données peut être réalisé.

A partir des données introduites lors de la première étape, une expression analytique de chaque surface va pouvoir être déterminée ou calculée pour chaque point du maillage (étape S3).

Les données issues de l'étape S2 vont permettre un calcul des temps moyens $<t_i>$ pour chacune des mesures effectuées (étape S4).

Les étapes S1 et S3 d'une part, S2 et S34 d'autre part, sont représentées en figure 9 comme sensiblement réalisées simultanément, mais ce n'est pas nécessaire, quoique souhaitable en vue d'un déroulement le plus rapide possible du procédé.

A partir des données de calcul des étapes S3 et S4, on recherche les points du maillage tels que $\xi_i = <t_i>$, pour tout i, à X% près (étape S5), ce qui signifie $|\xi_i - <t_i>| \leq X/100$. L'utilisateur peut lui-même fixer la précision X souhaitée.

Les résultats (l'intersection des surfaces entre elle, et éventuellement chacune des surfaces individuellement) peuvent ensuite être visualisés (étape S6).

Au besoin, les calculs peuvent être réitérés en fixant une valeur de X supérieure (auquel cas on réduit la précision) ou inférieure (auquel cas on accroît la précision) à la valeur précédente (étape S7).

## Revendications

1. Procédé de localisation d'au moins un fluorophore (22) ou d'au moins un absorbeur dans un milieu diffusant (20), à l'aide d'un dispositif comprenant une source (8, 10) de rayonnement en impulsion apte à émettre un rayonnement d'excitation de ce fluorophore ou de cet absorbeur et des moyens (4, 12) de détection apte à mesurer un signal de fluorescence ($\Phi_{fluo}$) émis par ce fluorophore (22) ou un signal d'émission émis par cet absorbeur, le dispositif permettant une détection résolue en temps de ces signaux, le procédé étant **caractérisé en ce qu'**il comporte

   a) pour au moins 3 couples différents de positions de la source de rayonnement et des moyens de détection, au moins une excitation par un rayonnement provenant de la source (8) de rayonnement, et au moins une détection par les moyens (4) de détection du signal de fluorescence émis par ce fluorophore après cette excitation, ou du signal d'émission émis par cet absorbeur,
   b) pour chacun de ces couples, l'identification d'une surface sur laquelle le fluorophore ou l'absorbeur est situé, ou d'un volume comprenant cette surface et dans lequel le fluorophore ou l'absorbeur est situé, cette identification comportant le calcul d'un moment d'ordre n$\geq$1 dudit signal de fluorescence ou dudit signal d'émission, ou d'un moment d'une fonction fréquentielle dudit signal de fluorescence ou dudit signal d'émission,
   c) une estimation de la localisation du fluorophore ou l'absorbeur dans son milieu diffusant, par calcul de l'intersection des trois surfaces, et éventuellement d'un volume autour de cette intersection.

2. Procédé selon la revendication 1, dans lequel ledit milieu diffusant, environnant le fluorophore ou l'absorbeur, est de type infini ou de type semi-infini ou forme une tranche, limitée par deux surfaces parallèles, ou est de forme quelconque, sa surface extérieure étant discrétisée en une série de plans.

3. Procédé selon l'une des revendications 1 ou 2, dans lequel le milieu diffusant est de type infini, les surfaces ayant alors la forme d'ellipsoïdes.

4. Procédé selon l'une des revendications 1 à 3, l'étape b), comportant, pour chaque couple de position, un calcul d'un moment temporel normé d'une fonction temporelle du signal de fluorescence ou du signal d'émission par l'absorbeur, ou d'un moment normé d'une fonction fréquentielle, déduite de ladite fonction temporelle du signal de fluorescence ou du signal d'émission par l'absorbeur.

5. Procédé selon la revendication 4, la fonction fréquentielle étant déduite de ladite fonction temporelle par transformée de Fourier ou par transformée de Laplace ou par transformée de Mellin

6. Procédé selon l'une des revendications 1 à 5, dans lequel on réalise une excitation par la source (8) de rayonnement, et une détection par les moyens (4) de détection pour 4 couples différents de positions de la source de rayonnement et des moyens de détection.

7. Procédé selon la revendication 6, dans lequel, pour la localisation d'au moins un fluorophore (22) :

   - on sélectionne parmi les quatre couples de points, le couple de points, dit quatrième couple de points, pour lequel la durée moyenne d'arrivée des photons, depuis l'instant d'émission par l'émetteur à l'instant de réception par le récepteur, est la plus courte, et on calcule, pour ce couple de points, l'équation d'une surface sur laquelle le fluorophore est situé,

- et, pour chacun des trois autres couples de points, on calcule une première équation d'une première surface sur laquelle le fluorophore est situé, et on effectue une différence entre cette équation et celle associée au quatrième couple, pour obtenir une deuxième équation d'une deuxième surface indépendante de la durée de vie du fluorophore

**8.** Procédé selon l'une des revendications 1 à 6, dans lequel, pour la localisation d'au moins un fluorophore (22), l'étape b), comporte, pour chaque couple de position, un calcul d'une équation de chaque surface, indépendante de la durée de vie $\tau$ du fluorophore

**9.** Procédé selon la revendication 8, ladite équation indépendante de la durée de vie $\tau$ résultant de la différence entre le temps moyen mesuré ou calculé pour chaque couple de position et le temps moyen mesuré ou calculé pour un quatrième couple de position.

**10.** Procédé selon la revendication 9, le quatrième couple de position étant un couple pour lequel le signal de fluorescence a un temps moyen mesuré ou un temps moyen calculé inférieur à celui des 3 couples de position.

**11.** Procédé selon l'une des revendications 1 à 10, la source de rayonnement étant l'extrémité d'une fibre optique (10) qui amène la lumière d'excitation dans le milieu et/ou les moyens de détection étant l'extrémité d'une fibre optique (12) qui prélève une partie de la lumière d'émission.

**12.** Dispositif pour la localisation d'au moins un fluorophore ou d'au moins un absorbeur dans un milieu diffusant, comportant :

    - une source (8, 10) de rayonnement en impulsion apte à émettre un rayonnement d'excitation,
    - des moyens (4, 12) de détection apte à mesurer un signal de fluorescence ($\Phi_{fluo}$) émis par ce fluorophore (22) ou un signal d'émission émis par cet absorbeur, le dispositif permettant une détection résolue en temps de ces signaux, le dispositif étant **caractérisé en ce qu'**il comporte
    - des moyens (24) de calcul, pour:

        a) pour chaque couple de points parmi au moins 3 couples différents de positions de la source de rayonnement et des moyens de détection, réaliser une calcul ou une détermination, comportant le calcul d'un moment d'ordre n$\geq$1 dudit signal de fluorescence ou dudit signal d'émission, après une excitation réalisée par un rayonnement provenant de la source (8) de rayonnement, et une détection par les moyens (4) de détection des signaux émis par le fluorophore ou l'absorbeur après cette excitation, d'une surface sur laquelle le fluorophore ou l'absorbeur est situé, ou un volume comprenant cette surface et dans lequel le fluorophore ou l'absorbeur est situé,
        b) et pour estimer la localisation du fluorophore ou de l'absorbeur dans son milieu environnant, par calcul de l'intersection des trois surfaces ou d'un volume autour de cette intersection.

**13.** Dispositif selon l'une des revendications 12, les moyens de calcul permettant de calculer, pour chaque couple de position, un moment temporel normé d'une fonction temporelle, ou un moment normé d'une fonction fréquentielle, déduite de ladite fonction temporelle d'émission.

**14.** Dispositif selon l'une des revendications 12 ou 13, lesdits moyens de détection comportant des moyens de type TCSPC ou des moyens de type caméra.

**15.** Dispositif selon l'une des revendications 12 à 14, comportant en outre des moyens (27) de représentation visuelle ou graphique de la localisation du fluorophore ou de la molécule absorbante.

**Patentansprüche**

**1.** Verfahren zur Lokalisierung wenigstens eines Fluorophors (22) oder wenigstens eines Absorbers in einem ausstrahlenden Medium (20) mit Hilfe einer Vorrichtung, umfassend eine Pulsstrahlungsquelle (8, 10), die dazu ausgelegt ist, eine Strahlung zur Anregung dieses Fluorophors oder dieses Absorbers zu emittieren, sowie Erfassungsmittel (4, 12), die dazu ausgelegt sind, ein Fluoreszenzsignal ($\Phi_{fluo}$) zu messen, das von diesem Fluorophor (22) emittiert wird, oder ein Emissionssignal, das von diesem Absorber emittiert wird, wobei die Vorrichtung eine zeitaufgelöste Erfassung dieser Signale erlaubt, wobei das Verfahren **dadurch gekennzeichnet ist, dass** es umfaßt:

a) für wenigstens drei verschiedene Paare von Positionen der Strahlungsquelle und der Erfassungsmittel, wenigstens eine Anregung durch eine Strahlung, die von der Strahlungsquelle (8) stammt, sowie wenigstens eine Erfassung durch die Mittel (4) zur Erfassung des Fluoreszenzsignals, das durch diesen Fluorophor nach dieser Anregung emittiert wird, oder des Emissionssignals, das durch diesen Absorber emittiert wird,

b) für jedes dieser Paare die Identifikation einer Fläche, auf der der Fluorophor oder der Absorber angeordnet ist, oder eines Volumens, welches diese Fläche umfaßt und in dem der Fluorophor oder der Absorber angeordnet ist, wobei diese Identifikation die Berechnung eines Moments der Ordnung $n \geq 1$ des Fluoreszenzsignals oder des Emissionssignals umfaßt, oder eines Moments einer Frequenzfunktion des Fluoreszenzsignals oder des Emissionssignals,

c) eine Abschätzung der Lokalisierung des Fluorophors oder des Absorbers in seinem ausstrahlenden Medium durch Berechnung des Schnitts der drei Flächen sowie gegebenenfalls eines Volumens um diesen Schnitt herum.

2. Verfahren nach Anspruch 1, bei dem das ausstrahlende Medium, welches den Fluorophor oder den Absorber umgibt, vom unendlichen Typ oder vom halbunendlichen Typ ist oder eine Scheibe bildet, die durch zwei parallele Flächen begrenzt ist, oder von beliebiger Form ist, wobei seine Außenfläche in einer Reihe von Ebenen diskretisiert ist.

3. Verfahren nach einem der Ansprüche 1 oder 2, bei dem das ausstrahlende Medium vom unendlichen Typ ist, wobei die Flächen dann die Form von Ellipsoiden haben.

4. Verfahren nach einem der Ansprüche 1 bis 3, wobei der Schritt b) für jedes Paar von Positionen eine Berechnung eines normierten Zeitmoments einer Zeitfunktion des Fluoreszenzsignals oder des Absorberemissionssignals umfaßt, oder eines normierten Moments einer Frequenzfunktion, abgeleitet aus der Zeitfunktion des Fluoreszenzsignals oder des Absorberemissionssignals.

5. Verfahren nach Anspruch 4, wobei die Frequenzfunktion abgeleitet ist aus der Zeitfunktion durch Fourier-Transformation oder durch Laplace-Transformation oder durch Mellin-Transformation.

6. Verfahren nach einem der Ansprüche 1 bis 5, bei dem man eine Anregung durch die Strahlungsquelle (8) und eine Erfassung durch die Erfassungsmittel (4) für vier verschiedene Paare von Positionen der Strahlungsquelle und der Erfassungsmittel realisiert.

7. Verfahren nach Anspruch 6, bei dem man für die Lokalisierung wenigstens eines Fluorophors (22):

- aus den vier Paaren von Punkten dasjenige Paar von Punkten auswählt, genannt viertes Paar von Punkten, für das die mittlere Ankunftsdauer der Photonen ab dem Zeitpunkt der Emission durch den Aussender bis zum Zeitpunkt des Empfangs durch den Empfänger am kürzesten ist, und man für dieses Paar von Punkten die Gleichung einer Fläche berechnet, auf der der Fluorophor angeordnet ist,

- und für jedes der drei anderen Paare von Punkten eine erste Gleichung einer ersten Fläche berechnet, auf der der Fluorophor angeordnet ist, und man eine Differenz bildet zwischen dieser Gleichung und jener, die dem vierten Paar zugeordnet ist, um eine zweite Gleichung einer zweiten Fläche zu erhalten, die unabhängig ist von der Lebensdauer des Fluorophors.

8. Verfahren nach einem der Ansprüche 1 bis 6, bei dem der Schritt b) für die Lokalisierung wenigstens eines Fluorophors (22) für jedes Paar von Positionen eine Berechnung einer Gleichung jeder Oberfläche umfaßt, die unabhängig ist von der Lebensdauer $\tau$ des Fluorophors.

9. Verfahren nach Anspruch 8, wobei die Gleichung, die unabhängig ist von der Lebensdauer $\tau$, aus der Differenz zwischen der gemessenen oder berechneten mittleren Zeit für jedes Paar von Positionen und der gemessenen oder berechneten mittleren Zeit für ein viertes Paar von Positionen resultiert.

10. Verfahren nach Anspruch 9, wobei das vierte Paar von Positionen ein Paar ist, für das das Fluoreszenzsignal eine gemessene mittlere Zeit oder eine berechnete mittlere Zeit kleiner als jene der drei Paare von Positionen hat.

11. Verfahren nach einem der Ansprüche 1 bis 10, wobei die Strahlungsquelle das Ende einer optischen Faser (10) ist, die das Anregungslicht in das Medium bringt, und/oder wobei die Erfassungsmittel das Ende einer optischen Faser (12) sind, die einen Teil des Emissionslichts entnimmt.

**12.** Vorrichtung zur Lokalisierung wenigstens eines Fluorophors oder wenigstens eines Absorbers in einem ausstrahlenden Medium, umfassend:

- eine Pulsstrahlungsquelle (8, 10), die dazu ausgelegt ist, eine Anregungsstrahlung zu emittieren,
- Erfassungsmittel (4, 12), die dazu ausgelegt sind, ein Fluoreszenzsignal ($\Phi_{fluo}$) zu messen, das durch diesen Fluorophor (22) emittiert wird, oder ein Emissionssignal, das durch diesen Absorber emittiert wird,

wobei die Vorrichtung eine zeitaufgelöste Erfassung dieser Signale ermöglicht, wobei die Vorrichtung **dadurch gekennzeichnet ist, dass** sie Rechenmittel (24) umfaßt, um:

a) für jedes Paar von Punkten aus wenigstens drei verschiedenen Paaren von Positionen der Strahlungsquelle und der Erfassungsmittel eine Berechnung oder eine Bestimmung zu realisieren, umfassend die Berechnung eines Moments der Ordnung n ≥ 1 des Fluoreszenzsignals oder des Emissionssignals nach einer Anregung, die durch eine Strahlung realisiert ist, welche von der Strahlungsquelle (8) stammt, sowie einer Erfassung der durch den Fluorophor oder den Absorber emittierten Signale nach dieser Anregung durch die Erfassungsmittel (4), einer Fläche, auf der der Fluorophor oder der Absorber angeordnet ist, oder eines Volumens, das diese Fläche umfaßt und in dem der Fluorophor oder der Absorber angeordnet ist,
b) und zum Abschätzen der Lokalisierung des Fluorophors oder des Absorbers in seinem umgebenden Medium durch Berechnung des Schnitts der drei Flächen oder eines Volumens um diesen Schnitt herum.

**13.** Vorrichtung nach einem der Ansprüche 12, wobei die Rechenmittel es ermöglichen, für jedes Paar von Positionen ein normiertes Zeitmoment einer Zeitfunktion zu berechnen oder ein normiertes Moment einer Frequenzfunktion, die aus der Emissionszeitfunktion abgeleitet ist.

**14.** Vorrichtung nach einem der Ansprüche 12 oder 13, wobei die Erfassungsmittel Mittel vom Typ TCSPC oder Mittel vom Kameratyp umfassen.

**15.** Vorrichtung nach einem der Ansprüche 12 bis 14, ferner umfassend Mittel (27) zur visuellen oder graphischen Darstellung der Lokalisierung des Fluorophors oder des absorbierenden Moleküls.

**Claims**

**1.** Method of localising at least one fluorophore (22) or at least one absorber in a scattering medium (20), by means of a device comprising a pulse radiation source (8, 10) suited to emitting an excitation radiation of this fluorophore or this molecule and detection means (4, 12) suited to measuring a fluorescence signal emitted by this fluorophore (22) or an emission signal emitted by this absorber, said device allowing a time resolved detection of these signals, the method being **characterized in that** it comprises:

a) for at least 3 different pairs of positions of the radiation source and detection means, at least one excitation by a radiation coming from the radiation source (8), and at least one detection by the means (4) of detecting the fluorescence signal emitted by this fluorophore after this excitation, or of the emission signal emitted by this absorber,
b) for each of these pairs, the identification of a surface on which the fluorophore or the absorber is situated, or of a volume comprising this surface and in which the fluorophore or the absorber is situated, this identification comprising the calculation of a moment of order n≥1 of said fluorescence signal or of said emission signal or of a frequency function of said fluorescence signal or of said emission signal,
c) an estimation of the localisation of the fluorophore or the absorber in its scattering medium, by calculation of the intersection of the three surfaces, and possibly of a volume around this intersection.

**2.** Method according to claim 1, wherein the diffusing medium, surrounding the fluorophore or the absorber is of infinite type or semi-infinite type or forms a slab, limited by two parallel surfaces, or is of any shape, its exterior surface being discretised into a series of planes.

**3.** Method according to any of claims 1 or 2, wherein the scattering medium is of infinite type, the surfaces then having the shape of ellipsoids.

**4.** Method according to any of claims 1 to 3, step b) comprising, for each position pair, a calculation of a normalised time moment, of a time function of the fluorescence signal or of the emission signal by the absorber, or of a normalised moment of a frequency function, deduced from said time function of the fluorescence signal or the emission signal by the absorber.

5. Method according to claim 4, the frequency function being deduced from said time function by Fourier transform or by Laplace transform or by Mellin transform.

6. Method according to any of claims 1 to 5, wherein an excitation is carried out by the radiation source (8), and a detection by the detection means (4) for 4 different pairs of positions of the radiation source and detection means.

7. Method according to claim 6, wherein, for the localisation of at least one fluorophore:

   - among the four pairs of points are selected the pair of points, known as fourth pair of points, for which the average arrival time of the photons, from the time of emission by the emitter to the time of reception by the receiver, is the shortest, and, for this pair of points, the equation of a surface on which the fluorophore is situated is calculated,
   - and, for each of the three other pairs of points, a first equation of a first surface on which the fluorophore is situated is calculated, and a difference is taken between this equation and that associated with the fourth pair, to obtain a second equation of a second surface independent of the lifetime of the fluorophore

8. Method according to any of claims 1 to 6, wherein, for the localisation of at least one fluorophore (22), step b) comprises, for each position pair, a calculation of an equation of each surface, independent of the lifetime of the fluorophore.

9. Method according to claim 8, said equation independent of $\tau$ resulting from the difference between the average time measured or calculated for each position pair and the average time measured or calculated for a fourth position pair.

10. Method according to claim 9, the fourth position pair being a pair for which the fluorescence signal has a measured average time or a calculated average time less than that of the 3 position pairs.

11. Method according to any of claims 1 to 10, the radiation source being the end of an optic fibre (10) which sends light into the medium and/or and the detection means being the end of an optic fibre (12), which samples a part of the emission light.

12. Device for the localisation of at least one fluorophore or at least one absorber in a scattering medium, comprising:

   - a pulse radiation source (8, 10) suited to emitting an excitation radiation,
   - detection means (4, 12) suited to measuring a fluorescence signal emitted by this fluorophore (22) or an emission signal emitted by this absorber,
   - the device allowing a time resolved detection of these signals,

   this device being **characterized in that** it comprises:

   calculation means for:

   a) for each pair of points among at least 3 different pairs of positions of the radiation source and detection means, carrying out a calculation or determining, comprising the calculation of a moment of order n > 1 of said fluorescence signal or said emission signal, after an excitation carried out by a radiation coming from the radiation source (8), and a detection by the means (4) of detecting signals emitted by the fluorophore or the absorber after this excitation, of a surface on which the fluorophore or the absorber is situated, or a volume comprising this surface and in which the fluorophore or the absorber is situated,
   b) and for estimating the localisation of the fluorophore or the absorber in its surrounding medium, by calculation of the intersection of the three surfaces or of a volume around this intersection.

13. Device according to claim 12, the calculation means making it possible to calculate, for each position pair, a normalised time moment of a time function, or a normalised moment of a frequency function, deduced from said emission time function.

**14.** Device according to claim 12 or 13, said detection means comprising TCSPC type means or camera type means.

**15.** Device according to one of claims 12 to 14, further comprising means of visual or graphic representation of the localisation of the fluorophore or the absorbent molecule.

FIG.1A

FIG.1B

FIG.2A

FIG.2B

FIG.3

## FIG.4A

## FIG.4B

FIG.4C

FIG.4D

FIG.4E

FIG.4F

FIG.5A

FIG.5B

FIG.5C

FIG.5D

FIG.6A

FIG.6B

FIG.6C

FIG.7A

FIG.7B

FIG.7C

FIG.7D

Coupe en y = -0.040816 cm

FIG.8A

FIG.8B

Coupe en y = -0.040816 cm

Coupe en x = 0.65306cm

FIG.8C

FIG.8D

FIG.9

FIG.10A

FIG.10B

FIG.10C

FIG.10D

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006032151 A

- EP 1884765 A

**Littérature non-brevet citée dans la description**

- **D'AURÉLIE LAIDEVANT.** *Méthodes optiques résolues en temps pour la tomographie de fluorescence dans les milieux diffusants,* 05 Octobre 2006, 51
- **A.LAIDEVANT et al.** Effects of the surface boundary on the determination of the optical properties of a turbid medium with time resolved reflectance. *Applied Optics,* 2006, vol. 45 (19), 4756

- **D'AURÉLIE LAIDEVANT.** *Méthodes optiques résolues en temps pour la tomographie de fluorescence dans les milieux diffusants,* 05 Octobre 2006, 55-56
- **M.S.PATERSON et al.** time resolved reflectance and transmittance for the non invasive measurement of tissue optical properties. *Applied Optics,* vol. 28, 2331-2336
- **A.LAIDEVANT et al.** *Applied Optics,* 2006, vol. 45 (19), 4756